(19) Europäisches Patentamt / European Patent Office / Office européen des brevets

(11) EP 2 646 545 B1

(12) # EUROPÄISCHE PATENTSCHRIFT

(45) Veröffentlichungstag und Bekanntmachung des Hinweises auf die Patenterteilung:
**07.09.2016 Patentblatt 2016/36**

(21) Anmeldenummer: **11776417.5**

(22) Anmeldetag: **26.10.2011**

(51) Int Cl.:
***C12N 9/24*** *(2006.01)* ***C12N 15/62*** *(2006.01)*
***C12N 5/10*** *(2006.01)*

(86) Internationale Anmeldenummer:
**PCT/EP2011/068734**

(87) Internationale Veröffentlichungsnummer:
**WO 2012/055904 (03.05.2012 Gazette 2012/18)**

(54) **TEMPERATURSTABILE ß-PYRANOSIDASE**

TEMPERATURE-STABLE ß-PYRANOSIDASE

ß-PYRANOSIDASE RESITANTE À LA TEMPÉRATURE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.10.2010 DE 102010042910**

(43) Veröffentlichungstag der Anmeldung:
**09.10.2013 Patentblatt 2013/41**

(73) Patentinhaber: **Clariant Produkte (Deutschland) GmbH**
**65929 Frankfurt am Main (DE)**

(72) Erfinder:
- **REISINGER, Christoph**
  **81477 München (DE)**
- **QOURA, Farah**
  **81671 München (DE)**
- **KLIPPEL, Barbara**
  **20535 Hamburg (DE)**
- **ANTRANIKIAN, Garabed**
  **21218 Hittfeld-Waldesruh (DE)**

(74) Vertreter: **Silber, Anton et al**
**Clariant Produkte (Deutschland) GmbH**
**Patent & License Management**
**Lenbachplatz 6**
**80333 München (DE)**

(56) Entgegenhaltungen:
**WO-A1-2009/094187 WO-A2-2009/146464**

- **DATABASE UniProt [Online] 13. November 2007 (2007-11-13), "SubName: Full=Glycoside hydrolase family 3 domain protein;", XP002667902, gefunden im EBI accession no. UNIPROT:A8F439 Database accession no. A8F439**
- **VARGHESE ET AL: "Three-dimensional structure of a barley beta-D-glucan exohydrolase, a family 3 glycosyl hydrolase", STRUCTURE, CURRENT BIOLOGY LTD., PHILADELPHIA, PA, US, Bd. 7, Nr. 2, 15. Februar 1999 (1999-02-15), Seiten 179-190, XP005606887, ISSN: 0969-2126, DOI: 10.1016/S0969-2126(99)80024-0 in der Anmeldung erwähnt**
- **HENRISSAT B ET AL: "A scheme for designating enzymes that hydrolyse the polysaccharides in the cell walls of plants", FEBS LETTERS, ELSEVIER, AMSTERDAM, NL, Bd. 425, Nr. 2, 27. März 1998 (1998-03-27) , Seiten 352-354, XP004262144, ISSN: 0014-5793, DOI: 10.1016/S0014-5793(98)00265-8 in der Anmeldung erwähnt**
- **ANDREWS K T ET AL: "Fervidobacterium gondwanense sp. nov., a new thermophilic anaerobic bacterium isolated from nonvolcanically heated geothermal waters of the Great Artesian Basin of Australia.", INTERNATIONAL JOURNAL OF SYSTEMATIC BACTERIOLOGY JAN 1996 LNKD- PUBMED:8573506, Bd. 46, Nr. 1, Januar 1996 (1996-01), Seiten 265-269, XP002667903, ISSN: 0020-7713**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 646 545 B1

SEQUENCE LISTING

<110> Sued-Chemie

<120> Temperaturstabile beta-Pyranosidase

<130> HE 150704

<160> 4

<170> PatentIn version 3.4

<210> 1
<211> 775
<212> PRT
<213> Fervidobacterium gondwanense

<400> 1

```
Met Glu Ile Tyr Lys Asp Ser Ser Lys Pro Ile Glu Leu Arg Val Glu
1               5                   10                  15

Asp Leu Leu Ser Arg Met Thr Leu Glu Glu Lys Val Ser Gln Leu Gly
            20                  25                  30

Ser Val Trp Ser Tyr Gln Leu Leu Asp Glu Asn Gly Asn Phe Asp Glu
        35                  40                  45

Gly Lys Ala Phe Glu Leu Leu Lys Asp Gly Ile Gly Gln Ile Ser Arg
    50                  55                  60

Pro Gly Gly Ala Thr Asn Phe Gln Pro Glu Glu Val Ala Gln Phe Asp
65                  70                  75                  80

Asn Lys Val Gln Lys Phe Leu Ile Glu Asn Thr Arg Leu Gly Ile Pro
                85                  90                  95

Ala Leu Met His Glu Glu Cys Leu Thr Gly Tyr Met Gly Leu Asn Gly
            100                 105                 110

Ser Asn Phe Pro Val Pro Ile Ala Met Ala Ser Thr Trp Glu Pro Glu
        115                 120                 125

Leu Val Lys Glu Val Thr Lys Val Ile Arg Gln Glu Met Arg Asn Met
    130                 135                 140

Gly Ile His Gln Gly Leu Ala Pro Val Leu Asp Val Ala Arg Asp Pro
145                 150                 155                 160

Arg Trp Gly Arg Val Glu Glu Thr Phe Gly Glu Ser Pro Tyr Leu Val
                165                 170                 175
```

```
Ala Ser Met Gly Cys Ala Tyr Val Glu Gly Leu Gln Gly Glu Asp Leu
            180                 185                 190

Lys Asp Gly Val Ile Ala Thr Thr Lys His Phe Val Gly Tyr Ser Ala
        195                 200                 205

Ser Glu Gly Gly Arg Asn Trp Ala Pro Thr Asn Ile Pro Pro Arg Glu
    210                 215                 220

Leu Arg Glu Ile Phe Met Phe Pro Phe Glu Ala Ala Val Lys Val Ala
225                 230                 235                 240

Lys Val Gly Ser Val Met Asn Ser Tyr Ser Glu Ile Asp Gly Val Pro
                245                 250                 255

Leu Ala Ala Ser Arg Glu Leu Leu Thr Asp Val Leu Arg Lys Glu Trp
            260                 265                 270

Gly Phe Asp Gly Leu Val Val Ser Asp Tyr Phe Ser Val Lys Leu Ile
        275                 280                 285

His Glu His His Lys Leu Ala Arg Asp Lys Ala Glu Ala Ala Lys Tyr
    290                 295                 300

Ala Leu Glu Ala Gly Ile Asp Val Glu Leu Pro Asn Thr Asp Cys Tyr
305                 310                 315                 320

Ala His Val Leu Asp Leu Val Lys Ser Gly Val Ile Pro Glu Lys Leu
                325                 330                 335

Leu Asp Gln Thr Val Arg Arg Ile Leu Lys Met Lys Phe Lys Leu Gly
            340                 345                 350

Leu Phe Asp Lys Pro Tyr Val Glu Pro Ser Lys Ala Lys Val Val Lys
        355                 360                 365

Asn Thr Glu Leu Ala Leu Glu Val Ala Arg Lys Ser Ile Val Leu Leu
    370                 375                 380

Lys Asn Asp Gly Ile Leu Pro Leu Lys Lys Asp Met Lys Val Ala Leu
385                 390                 395                 400

Ile Gly Pro Asn Ala Ala Asp Val Arg Asn Met Leu Gly Asp Tyr Met
                405                 410                 415

Tyr Leu Ala His Ile Lys Ile Met Leu Glu Asn Val Asn Leu Ala Phe
            420                 425                 430
```

```
Asp Ala Pro Lys Phe Asn Leu Ser Ser Val Lys Lys Ser Val Glu Glu
        435                 440                 445

Ser Met Asn Lys Ile Lys Ser Ile Glu Met Leu Leu Lys Glu Glu Ser
    450                 455                 460

Val Gln Phe Thr Tyr Ala Lys Gly Cys Asp Val Leu Gly Asp Ser Lys
465                 470                 475                 480

Glu Gly Phe Asn Glu Ala Leu Lys Ala Val Glu Asn Ser Asp Val Ala
                485                 490                 495

Ile Val Val Val Gly Asp Arg Ser Gly Leu Thr Met Asp Cys Thr Thr
            500                 505                 510

Gly Glu Ser Arg Asp Ser Ala Asn Leu Lys Leu Pro Gly Val Gln Glu
        515                 520                 525

Glu Leu Ile Ile Glu Val Ser Lys Val Gly Lys Pro Val Val Leu Ala
    530                 535                 540

Leu Leu Asn Gly Arg Pro Tyr Ser Leu Thr Arg Val Val Asp Lys Val
545                 550                 555                 560

Ser Ala Ile Val Glu Ala Trp Leu Pro Gly Glu Ile Gly Ala Lys Ala
                565                 570                 575

Ile Val Asp Val Leu Tyr Gly Lys Val Asn Pro Ser Gly Lys Leu Pro
            580                 585                 590

Met Thr Phe Pro Arg Ser Ala Gly Gln Ile Pro Leu Phe His Tyr Phe
        595                 600                 605

Lys Pro Ser Gly Gly Arg Ser Ser Trp His Gly Asp Tyr Val Asp Glu
    610                 615                 620

Ser Val Lys Pro Leu Phe Pro Phe Gly His Gly Leu Ser Tyr Thr Asn
625                 630                 635                 640

Phe Asp Tyr Ser Gly Leu Glu Ile Ser Pro Ser Lys Val Pro Met Ala
                645                 650                 655

Gly Ser Val Glu Ile Ser Leu Tyr Val Glu Asn Thr Gly Glu Val Glu
            660                 665                 670

Gly Glu Glu Val Val Gln Leu Tyr Ile Gly Arg Glu Cys Ala Ser Val
        675                 680                 685
```

Thr Arg Pro Val Lys Glu Leu Lys Gly Phe Ala Lys Val Asn Leu Lys
690 695 700

Pro Gly Glu Lys Arg Lys Val Leu Phe Asn Leu His Thr Asp Val Leu
705 710 715 720

Ala Phe Tyr Gly Arg Asp Met Lys Leu Cys Val Glu Pro Gly Val Tyr
725 730 735

Asn Val Met Ile Gly Ser Ser Ser Asp Asp Ile Arg Leu Lys Gly Ser
740 745 750

Phe Glu Val Asp Gly Met Arg Arg Glu Val Phe Glu Asp Arg Val Phe
755 760 765

Phe Thr Lys Val Tyr Thr Phe
770 775

<210> 2
<211> 625
<212> PRT
<213> artificial

<220>
<223> segment consisting of amino acid residues 13 to 638 of SEQ ID NO 1

<400> 2

Arg Val Glu Asp Leu Leu Ser Arg Met Thr Leu Glu Glu Lys Val Ser
1 5 10 15

Gln Leu Gly Ser Val Trp Ser Tyr Gln Leu Leu Asp Glu Asn Gly Asn
20 25 30

Phe Asp Glu Gly Lys Ala Phe Glu Leu Leu Lys Asp Gly Ile Gly Gln
35 40 45

Ile Ser Arg Pro Gly Gly Ala Thr Asn Phe Gln Pro Glu Glu Val Ala
50 55 60

Gln Phe Asp Asn Lys Val Gln Lys Phe Leu Ile Glu Asn Thr Arg Leu
65 70 75 80

Gly Ile Pro Ala Leu Met His Glu Glu Cys Leu Thr Gly Tyr Met Gly
85 90 95

Leu Asn Gly Ser Asn Phe Pro Val Pro Ile Ala Met Ala Ser Thr Trp
100 105 110

```
Glu Pro Glu Leu Val Lys Glu Val Thr Lys Val Ile Arg Gln Glu Met
        115                 120                 125

Arg Asn Met Gly Ile His Gln Gly Leu Ala Pro Val Leu Asp Val Ala
    130                 135                 140

Arg Asp Pro Arg Trp Gly Arg Val Glu Glu Thr Phe Gly Glu Ser Pro
145                 150                 155                 160

Tyr Leu Val Ala Ser Met Gly Cys Ala Tyr Val Glu Gly Leu Gln Gly
                165                 170                 175

Glu Asp Leu Lys Asp Gly Val Ile Ala Thr Thr Lys His Phe Val Gly
            180                 185                 190

Tyr Ser Ala Ser Glu Gly Gly Arg Asn Trp Ala Pro Thr Asn Ile Pro
        195                 200                 205

Pro Arg Glu Leu Arg Glu Ile Phe Met Phe Pro Phe Glu Ala Ala Val
    210                 215                 220

Lys Val Ala Lys Val Gly Ser Val Met Asn Ser Tyr Ser Glu Ile Asp
225                 230                 235                 240

Gly Val Pro Leu Ala Ala Ser Arg Glu Leu Leu Thr Asp Val Leu Arg
                245                 250                 255

Lys Glu Trp Gly Phe Asp Gly Leu Val Val Ser Asp Tyr Phe Ser Val
            260                 265                 270

Lys Leu Ile His Glu His His Lys Leu Ala Arg Asp Lys Ala Glu Ala
        275                 280                 285

Ala Lys Tyr Ala Leu Glu Ala Gly Ile Asp Val Glu Leu Pro Asn Thr
    290                 295                 300

Asp Cys Tyr Ala His Val Leu Asp Leu Val Lys Ser Gly Val Ile Pro
305                 310                 315                 320

Glu Lys Leu Leu Asp Gln Thr Val Arg Arg Ile Leu Lys Met Lys Phe
                325                 330                 335

Lys Leu Gly Leu Phe Asp Lys Pro Tyr Val Glu Pro Ser Lys Ala Lys
            340                 345                 350

Val Val Lys Asn Thr Glu Leu Ala Leu Glu Val Ala Arg Lys Ser Ile
```

```
            355                 360                 365

Val Leu Leu Lys Asn Asp Gly Ile Leu Pro Leu Lys Lys Asp Met Lys
    370                 375                 380

Val Ala Leu Ile Gly Pro Asn Ala Ala Asp Val Arg Asn Met Leu Gly
385                 390                 395                 400

Asp Tyr Met Tyr Leu Ala His Ile Lys Ile Met Leu Glu Asn Val Asn
                405                 410                 415

Leu Ala Phe Asp Ala Pro Lys Phe Asn Leu Ser Ser Val Lys Lys Ser
            420                 425                 430

Val Glu Glu Ser Met Asn Lys Ile Lys Ser Ile Glu Met Leu Leu Lys
        435                 440                 445

Glu Glu Ser Val Gln Phe Thr Tyr Ala Lys Gly Cys Asp Val Leu Gly
    450                 455                 460

Asp Ser Lys Glu Gly Phe Asn Glu Ala Leu Lys Ala Val Glu Asn Ser
465                 470                 475                 480

Asp Val Ala Ile Val Val Val Gly Asp Arg Ser Gly Leu Thr Met Asp
                485                 490                 495

Cys Thr Thr Gly Glu Ser Arg Asp Ser Ala Asn Leu Lys Leu Pro Gly
            500                 505                 510

Val Gln Glu Glu Leu Ile Ile Glu Val Ser Lys Val Gly Lys Pro Val
        515                 520                 525

Val Leu Ala Leu Leu Asn Gly Arg Pro Tyr Ser Leu Thr Arg Val Val
    530                 535                 540

Asp Lys Val Ser Ala Ile Val Glu Ala Trp Leu Pro Gly Glu Ile Gly
545                 550                 555                 560

Ala Lys Ala Ile Val Asp Val Leu Tyr Gly Lys Val Asn Pro Ser Gly
                565                 570                 575

Lys Leu Pro Met Thr Phe Pro Arg Ser Ala Gly Gln Ile Pro Leu Phe
            580                 585                 590

His Tyr Phe Lys Pro Ser Gly Gly Arg Ser Ser Trp His Gly Asp Tyr
        595                 600                 605
```

```
Val Asp Glu Ser Val Lys Pro Leu Phe Pro Phe Gly His Gly Leu Ser
    610                     615                     620

Tyr
625
```

<210> 3
<211> 2328
<212> DNA
<213> Fervidobacterium gondwanense

<400> 3

```
atggagatat ataaggattc ttctaagcct attgaattga gagttgagga cctgctttcg     60
agaatgacgc tggaagaaaa ggttagccag ctcggttctg tttggagtta tcaattactc    120
gacgagaacg ggaatttcga tgaagggaaa gcatttgagc tgttgaagga cggcataggt    180
caaatctcaa gaccgggtgg agcaacaaac tttcaacccg aagaagttgc tcaattcgac    240
aataaagtac agaaattctt gatagaaaat acgagacttg gaataccagc attaatgcac    300
gaagaatgcc taactggata tatgggactc aacggttcga attttcctgt gcctatcgcg    360
atggcgagta cgtgggagcc tgaactggta aaggaagtta cgaaagtgat aaggcaagag    420
atgaggaata tgggaattca ccaagggctc gctcctgtac ttgacgttgc aagggaccca    480
agatggggaa gggttgagga aacattcgga gagtcgcctt atcttgtcgc aagtatgggg    540
tgtgcttatg tcgaaggtct gcagggagaa gacttgaaag acggagtcat tgccactaca    600
aagcactttg tcggttacag cgcgtctgaa ggagggcgga actgggctcc aactaacatt    660
ccgccacgcg agttgagaga gatcttcatg ttcccattcg aagctgccgt gaaagtagcg    720
aaggttggtt ctgttatgaa ttcgtacagc gagatagacg gagtgcctct tgccgcctcc    780
agagagcttt taacagatgt gctgaggaaa gaatggggat ttgatggact cgttgtctct    840
gattatttct cggtgaagtt gatccacgaa catcacaaat tagcaaggga taaagccgaa    900
gcagcaaaat acgcccttga agcgggaata gatgtggaat taccaaatac cgattgctat    960
gcacatgtct tagacctggt gaaaagcggg gttattccgg aaaagttgtt agatcaaact   1020
gttagaagga ttttgaagat gaaatttaaa ttgggcttgt tcgataagcc gtacgtagaa   1080
ccttcaaaag cgaaggttgt aaaaaacaca gagcttgctc ttgaagttgc aagaaagtca   1140
atagtgctcc ttaagaacga cggtatcttg ccattgaaaa aagatatgaa agtagcactc   1200
ataggaccaa atgcggcgga tgtcaggaac atgctcggtg attacatgta cttagcacat   1260
ataaaaataa tgctcgagaa cgttaacttg gcttttgatg cgcctaagtt caatctttca   1320
agcgtgaaga aatcggttga ggagagcatg aacaagatca agagtataga gatgttgctc   1380
aaagaagaaa gtgttcaatt cacatacgcg aaaggttgcg atgtcttggg agattcgaaa   1440
gaagggttca acgaggcgct caaagcagtc gagaatagcg atgtggcgat agttgtagtt   1500
```

```
ggtgataggt caggtttgac catggattgc acaacgggtg agtcgaggga cagcgcgaat   1560
ttgaagttgc ccggagttca ggaagaactt atcattgagg tttcaaaggt tggcaagcct   1620
gtggtgctcg ctttgctgaa tggcagacct tattctttaa ccagggttgt agacaaggtt   1680
tctgctattg ttgaggcgtg gttgccaggt gagattggtg caaaggcaat agtcgatgtg   1740
ctttacggca aagtcaatcc atccggtaaa cttccaatga ctttcccgag aagtgctggt   1800
cagattccgc tcttccacta cttcaaaccg tctggcggaa ggtccagttg gcatggtgac   1860
tacgttgacg agagtgtcaa accactattc ccgttcggtc acggactttc gtatactaat   1920
ttcgattaca gcgggttaga aatctctcca tcaaaagtgc caatggcagg aagcgtcgaa   1980
atttcacttt atgtggagaa cactggtgaa gttgaaggcg aagaggttgt gcagctttac   2040
atcggaagag aatgtgcctc agtaactcgg ccagttaagg aactgaaagg ttttgcgaag   2100
gtgaatttga aacctggcga aaagaggaaa gttttgttca atctccacac ggacgtgctc   2160
gcgttctacg gacgcgatat gaagctctgc gttgagcctg ggtttacaa cgtcatgatt   2220
ggaagttctt cggatgatat aaggcttaaa ggaagttttg aggtagatgg aatgagaaga   2280
gaggtatttg aagatagagt attctttaca aaagtttaca ctttctaa                2328
```

<210> 4
<211> 630
<212> PRT
<213> Hordeum vulgare

<300>
<301> Varghese et al.
<303> Structure
<304> 7
<305> 2
<306> 179-190
<307> 1999

<400> 4

```
Met Ala Leu Leu Thr Ala Pro Ala Val Phe Ala Ala Leu Leu Leu Phe
1               5                   10                  15

Trp Ala Val Leu Gly Gly Thr Asp Ala Asp Tyr Val Leu Tyr Lys Asp
            20                  25                  30

Ala Thr Lys Pro Val Glu Asp Arg Val Ala Asp Leu Leu Gly Arg Met
        35                  40                  45

Thr Leu Ala Glu Lys Ile Gly Gln Met Thr Gln Ile Glu Arg Leu Val
    50                  55                  60

Ala Thr Pro Asp Val Leu Arg Asp Asn Phe Ile Gly Ser Leu Leu Ser
65                  70                  75                  80
```

```
Gly Gly Gly Ser Val Pro Arg Lys Gly Ala Thr Ala Lys Glu Trp Gln
                85                  90                  95

Asp Met Val Asp Gly Phe Gln Lys Ala Cys Met Ser Thr Arg Leu Gly
            100                 105                 110

Ile Pro Met Ile Tyr Gly Ile Asp Ala Val His Gly Gln Asn Asn Val
        115                 120                 125

Tyr Gly Ala Thr Ile Phe Pro His Asn Val Gly Leu Gly Ala Thr Arg
    130                 135                 140

Asp Pro Tyr Leu Val Lys Arg Ile Gly Glu Ala Thr Ala Leu Glu Val
145                 150                 155                 160

Arg Ala Thr Gly Ile Gln Tyr Ala Phe Ala Pro Cys Ile Ala Val Cys
                165                 170                 175

Arg Asp Pro Arg Trp Gly Arg Cys Tyr Glu Ser Tyr Ser Glu Asp Arg
            180                 185                 190

Arg Ile Val Gln Ser Met Thr Glu Leu Ile Pro Gly Leu Gln Gly Asp
        195                 200                 205

Val Pro Lys Asp Phe Thr Ser Gly Met Pro Phe Val Ala Gly Lys Asn
    210                 215                 220

Lys Val Ala Ala Cys Ala Lys His Phe Val Gly Asp Gly Gly Thr Val
225                 230                 235                 240

Asp Gly Ile Asn Glu Asn Asn Thr Ile Ile Asn Arg Glu Gly Leu Met
                245                 250                 255

Asn Ile His Met Pro Ala Tyr Lys Asn Ala Met Asp Lys Gly Val Ser
            260                 265                 270

Thr Val Met Ile Ser Tyr Ser Ser Trp Asn Gly Val Lys Met His Ala
        275                 280                 285

Asn Gln Asp Leu Val Thr Gly Tyr Leu Lys Asp Thr Leu Lys Phe Lys
    290                 295                 300

Gly Phe Val Ile Ser Asp Trp Glu Gly Ile Asp Arg Ile Thr Thr Pro
305                 310                 315                 320

Ala Gly Ser Asp Tyr Ser Tyr Ser Val Lys Ala Ser Ile Leu Ala Gly
```

325 330 335

Leu Asp Met Ile Met Val Pro Asn Asn Tyr Gln Gln Phe Ile Ser Ile
340 345 350

Leu Thr Gly His Val Asn Gly Gly Val Ile Pro Met Ser Arg Ile Asp
355 360 365

Asp Ala Val Thr Arg Ile Leu Arg Val Lys Phe Thr Met Gly Leu Phe
370 375 380

Glu Asn Pro Tyr Ala Asp Pro Ala Met Ala Glu Gln Leu Gly Lys Gln
385 390 395 400

Glu His Arg Asp Leu Ala Arg Glu Ala Ala Arg Lys Ser Leu Val Leu
405 410 415

Leu Lys Asn Gly Lys Thr Ser Thr Asp Ala Pro Leu Leu Pro Leu Pro
420 425 430

Lys Lys Ala Pro Lys Ile Leu Val Ala Gly Ser His Ala Asp Asn Leu
435 440 445

Gly Tyr Gln Cys Gly Gly Trp Thr Ile Glu Trp Gln Gly Asp Thr Gly
450 455 460

Arg Thr Thr Val Gly Thr Thr Ile Leu Glu Ala Val Lys Ala Ala Val
465 470 475 480

Asp Pro Ser Thr Val Val Val Phe Ala Glu Asn Pro Asp Ala Glu Phe
485 490 495

Val Lys Ser Gly Gly Phe Ser Tyr Ala Ile Val Ala Val Gly Glu His
500 505 510

Pro Tyr Thr Glu Thr Lys Gly Asp Asn Leu Asn Leu Thr Ile Pro Glu
515 520 525

Pro Gly Leu Ser Thr Val Gln Ala Val Cys Gly Gly Val Arg Cys Ala
530 535 540

Thr Val Leu Ile Ser Gly Arg Pro Val Val Val Gln Pro Leu Leu Ala
545 550 555 560

Ala Ser Asp Ala Leu Val Ala Ala Trp Leu Pro Gly Ser Glu Gly Gln
565 570 575

```
Gly Val Thr Asp Ala Leu Phe Gly Asp Phe Gly Phe Thr Gly Arg Leu
            580                 585                 590

Pro Arg Thr Trp Phe Lys Ser Val Asp Gln Leu Pro Met Asn Val Gly
        595                 600                 605

Asp Ala His Tyr Asp Pro Leu Phe Arg Leu Gly Tyr Gly Leu Thr Thr
    610                 615                 620

Asn Ala Thr Lys Lys Tyr
625                 630
```

**Beschreibung**

**Hintergrund**

Die Nutzung nachwachsender Rohstoffe aus pflanzlicher Biomasse als alternative Energiequelle gewinnt angesichts sinkender Vorräte an fossilen Brennstoffen und steigender $CO_2$-Emissionen immer mehr an Bedeutung. Durch den kontinuierlich und rasant zunehmenden Energiebedarf der Industrie- und Schwellenländer müssen neben einer effizienteren Nutzung der fossilen Energieträger auch äquivalente Alternativen als Energiequellen gefunden werden (Lyko et al., 2009, J. Biotechnol. 142(1):78-86).

In den Mittelpunkt der Forschung rückt dabei das Konzept der Bioraffinerie, bei dem pflanzliche Biomasse sowohl als Energie- als auch als Rohstofflieferant genutzt wird. Das Ziel der Bioraffinerie ist die vollständige Nutzung nachwachsender Rohstoffe für die Herstellung von Chemikalien, Kraftstoffen und Energie. Als Ausgangsstoff der Lignocellulose-Bioraffinerie dient trockene Biomasse wie Stroh und Holz aus lignocellulosehaltigen Roh- und Reststoffen.

Ein besonderes Interesse gilt der Nutzung lignocellulosehaltiger Rohstoffe, die aus land- und forstwirtschaftlichen Rückständen wie z. B. Stroh und Holz stammen, da diese kostengünstig sind und nicht in Konkurrenz zur Nahrungs- und Futtermittelindustrie stehen (Lyko et al., 2009, J. Biotechnol. 142(1):78-86; Kumar et al., 2008, J. Ind. Microbiol. Biotechnol. 35(5):377-391; Peters, 2007, Adv. Biochem. Eng. Biotechnol. 105:1-30; Kamm et al., 2006, Biochem. Mol. Biol. Int. 44(2):283-292). Lignocellulose ist das mengenmäßig am häufigsten vorkommende Biopolymer der Erde und besteht aus Cellulose, Hemicellulose und Lignin. Der Anteil von Cellulose beträgt ca. 30-60%, der von Hemicellulose ca. 20-40% und der Lignin-Anteil liegt bei ca. 10-30%. Im Gegensatz zur Cellulose, die aus unverzweigten Glukoseeinheiten zusammengesetzt ist, besteht Hemicellulose aus Pentosen und Hexosen, die zusätzliche Verzweigungen aus Kohlenhydraten aufweisen können. Lignin hingegen ist ein Polymer aus phenolischen Molekülen (Peters, 2007, Adv. Biochem. Eng. Biotechnol. 105:1-30).

Die Verwendung von Lignocellulose in der Bioraffinerie erfordert die Umsetzung des Rohstoffs in verwertbare Zucker. Dafür wird die Lignocellulose durch mechanische, thermische und/oder chemische Methoden vorbehandelt, um die Cellulose und Hemicellulose für die anschließende enzymatische Hydrolyse besser zugänglich zu machen (Hendriks & Zeeman, 2009, Bioresour. Technol. 100(1):10-18). Bei der enzymatischen Hydrolyse spielen vor allem cellulolytische und xylanolytische Enzyme eine entscheidende Rolle. Diese zu den Glykosid-Hydrolasen gehörenden Enzyme sind in der Lage, die glykosidischen Bindungen in der Cellulose und der Hemicellulose zu spalten.

Neben dem Einsatz von cellulolytischen und xylanolytischen Enzymen aus mesophilen Mikroorganismen ist die Nutzung thermostabiler Enzyme von wachsendem Interesse, da sie sich für Prozesse eignen, die bei hohen Temperaturen ablaufen, wobei die Löslichkeit und somit die Zugänglichkeit des Substrats erhöht wird (Turner et al., 2007, Microb. Cell. Fact. 6:9). Zusätzlich zeichnen sich thermostabile Enzyme durch eine erhöhte Substratspezifität und Stabilität gegenüber Lösungsmitteln und Detergenzien aus (Viikari et al., 2007, Adv. Biochem. Eng. Biotechnol. 108:121-145; Antranikian G. (2008) in: Industrial relevance of thermophiles and their enzymes - Thermophiles - Biology and Technology at high temperatures, ed. Robb Fea (CRC Press, Taylor & Francis, Boca Raton), pp 113-160).

**Xylan**

Xylan, das zweithäufigste Polysaccharid in der Natur, bildet den Hauptbestandteil der Hemicellulose. Es ist innerhalb der Fibrille zwischen Cellulose und Lignin angeordnet und übernimmt eine wichtige Rolle beim Zusammenhalt der Mikrofibrillen. Xylane bestehen aus einem homopolymeren Rückgrat aus $\beta$-1,4-verknüpften Xylopyranoseeinheiten. Dieses liegt entweder linear und unsubstituiert vor, kann zusätzlich acetyliert oder mit Arabinosyl- und Glukuronopyranosylgruppen substituiert sein. Daher erfolgt eine Unterscheidung in Homoxylan, Arabinoxylan, Glukuronoxylan und Glukuronoarabinoxylan (Saha, 2003, J. Ind. Microbiol, Biotechnol. 30(5):279-291; Bergquist et al., 2001, Methods Enzymol 330:301-319; Kulkarni et al., 1999, FEMS Microbiol. Rev. 23(4):411-456). Meist liegen Xylane als komplexe, stark verzweigte Heteropolymere vor (Collins *et al.*, 2002).

Die komplette Hydrolyse von Xylanen zu Monosacchariden erfordert eine Vielzahl von Enzymen, die gemeinsam am Abbau beteiligt sind (Collins et al., 2005, FEMS Microbiol. Rev. 29(1):3-23; Bergquist et al., 2001, Methods Enzymol 330:301-319). Endoxylanasen spalten die glykosidischen Bindungen innerhalb des Xylan-Rückgrats und bilden dabei hauptsächlich kürzere Xylooligosaccharide, aber auch Xylose, Xylobiose und Xylotriose (Polizeli et al. 2005, Appl. Microbiol. Biotechnol. 67(5):577-591; Dwivedi et al., 1996, Appl. Microbiol. Biotechnol. 45(1-2):86-93). $\beta$-Xylosidasen spalten Xylose-Monomere vom nicht-reduzierenden Ende der Xylooligosaccharide und Xylobiose ab, wohingegen Xylan üblicherweise nicht als Substrat genutzt wird (Collins et al., 2005, FEMS Microbiol. Rev. 29(1):3-23; Polizeli et al., 2005, Appl. Microbiol. Biotechnol. 67(5):577-591). An der Freisetzung der Seitengruppen von heterogenen Xylanen sind weitere Enzyme wie $\alpha$-Arabinofuranosidasen, $\alpha$-Glukuronidasen und Acetylxylan-Esterasen beteiligt. $\alpha$-Arabinofuranosidasen spalten Arabinose von verzweigten Arabinoxylanen und Arabinanen ab, während $\alpha$-Glukuronidasen die $\alpha$-1,2-Bindungen zwischen dem $\beta$-Xylopyranosylrückgrat und der Glukuronsäure hydrolysieren. Acetyliertes Xylan wird durch Acetylxylan-Esterasen hydrolysiert, welche die Acetylgruppen des Xylans abspalten (Jaeger et al., 2006, Biokatalyse. Angewandte Mikrobiologie, ed. Antranikian G. Springer Verlag, Seiten 135-160). Xylanolytische Enzyme wurden sowohl in Pilzen und Bakterien als auch in Archaeen identifiziert.

**Glykosid-Hydrolasen**

Glykosid-Hydrolasen sind Enzyme, die glykosidische Bindungen zwischen Kohlenhydraten bzw. einem Kohlenhydrat und einem nicht Kohlenhydrat-haltigen Rest hydrolysieren. Sie spalten eine Vielzahl von $\alpha$- und $\beta$-glykosidische verknüpften Substraten und werden hinsichtlich ihrer Substratspezifität unterschieden. Eine Einteilung von Glykosid-Hydrolasen erfolgt aufgrund von Ähnlichkeiten der Aminosäuresequenz bzw. prozentualen Identitäten der Aminosäuresequenz in sogenannte Glykosid-Hydrolase-Familien (GH-Familien). Die Mitglieder einer Familie zeigen dabei neben einer ähnlichen Aminosäuresequenz eine ähnliche dreidimensionale Struktur und den gleichen Reaktionsmechanismus (Henrissat, 1991, Biochem. J. 280 (Pt 2):309-316).

Eine Liste der GH-Familien kann der CAZy-Datenbank ("Carbohydrate-Active enZymes") im Internet entnommen werden (Cantarel *et al*., 2009). Glykosid-Hydrolasen werden entsprechend ihres Reaktionsmechanismus in zwei Klassen unterteilt: (1) in Enzyme, die die glykosidischen Bindungen mit einer Umkehrung der Konfiguration des anomeren Kohlenstoffatoms spalten und (2) in Enzyme, welche die glykosidischen Bindungen unter Beibehaltung der anomeren Konfiguration hydrolysieren (Davies & Henrissat, 1995, Structure 3(9):853-859; McCarter & Withers, 1994, Curr. Opin. Struct Biol 4(6):885-892). In den meisten Glykosid-Hydrolasen sind Aspartat- und/oder Glutamatreste als katalytische Aminosäuren identifiziert worden, jedoch können auch andere Aminosäurereste an der Spaltung der glykosidischen Bindung beteiligt sein (Davies & Henrissat, 1995, Structure 3(9):853-859).

Glykosid-Hydrolasen bestehen aus einer katalytischen Domäne und können zusätzliche Kohlenhydrat-bindende Domänen enthalten. Diese sind über einen flexiblen Linker mit der katalytischen Domäne verbunden und ermöglichen den Enzymen die Bindung an das Substrat (Shoseyov *et al*., 2006; Boraston et al., 2004, Biochem. J. 382(Pt 3):769-781). Die Nomenklatur für Glykosid-Hydrolasen wurde durch Henrissat et al. (1998, FEBS Lett. 425(2):352-354) vereinheitlicht.

So erfolgt die Benennung der Enzyme und der dafür codierenden Gene durch Angabe des Substrats in Form von drei Buchstaben. Nach der Bezeichnung für das Substrat folgt die Nummer der GH-Familie, zu der das Enzym gehört, und ein Buchstabe, der die Reihenfolge angibt, in der die Enzyme identifiziert wurden. Zur Unterscheidung ähnlicher Enzyme aus verschiedenen Organismen wird eine Abkürzung für die Art vorangestellt.

**Endoxylanasen aus thermophilen Bakterien**

Es sind eine Reihe von Endoxylanasen aus thermophilen Bakterien bekannt, die anhand von Ähnlichkeiten der Aminosäuresequenzen der Aminosäuresequenzen in die Glykosid-Hydrolase-Familien 10, 11 und 43 nach Henrissat et al. (1998, FEBS Lett. 425(2):352-354) eingeordnet werden. Bakterien der Gattung *Caldicellulosiruptor* produzieren Endoxylanasen mit maximalen Aktivitäten bei Temperaturen von 65-70 °C und pH 5,5-6,5. Viele Endoxylanasen werden auch von dem anaeroben Bakterium *Clostridium* gebildet.

Die Enzyme XynC und XynX aus *C. thermocellum* zeigen zusätzlich zur Endoxylanase-Aktivität die Aktivität einer Endoglukanase (Jung et al., 1998, Biochem. Mol. Biol. Int. 44(2):283-292; Hayashi et al., 1997, J. Bacteriol. 179(13):4246-4253). Von den Endoxylanasen XynC und XynY ist bekannt, dass sie in Cellulosomen lokalisiert sind (Hayashi et al., 1997, J. Bacteriol. 179(13):4246-4253; Fontes et al., 1995, Biochem. J. 307 (Pt 1):151-158).

Die thermostabilsten Endoxylanasen werden von *Thermotoga maritima* und *T. neapolitana* produziert. Diese zeigen maximale Aktivitäten bei 85-105 °C und Halbwertszeiten von bis zu 22 h bei 90 °C bzw. 12 h bei 95 °C (Zverlov et al., 1996 Appl. Microbiol. Biotechnol. 45(1-2):245-247; Saul et al., 1995, Appl. Environ. Microbiol. 61(11):4110-4113; Winterhalter & Liebl, 1995, Appl. Environ. Microbiol. 61(5):1810-1815). Weitere Endoxylanasen werden von thermophilen Bakterien aus den Gattungen *Geobacillus*, *Rhodothermus, Thermoanaerobacterium* und *Thermobifida* gebildet.

**$\beta$-Xylosidasen aus thermophilen Bakterien**

Die bisher bekannten $\beta$-Xylosidasen werden von thermophilen Bakterien der Gattungen *Caldicellulosiruptor*, *Clostridium*, *Geobacillus*, *Thermoanaerobacter* und *Thermoanaerobacterium* gebildet. Ihre Einteilung erfolgt aufgrund von Ähnlichkeiten der Aminosäuresequenz in die Glykosid-Hydrolase-Familien 3, 39, 43 und 52 nach Henrissat et al. (1998, FEBS Lett. 425(2):352-354). Das anaerobe Bakterium *Clostridium stercorarium* produziert neben den $\beta$-Xylosidasen auch Endoxylanasen und $\alpha$-Arabinofuranosidasen und ist somit in der Lage, Arabinoxylan vollständig zu Xylose und Arabinan abbauen zu können (Adelsberger et al., 2004, Microbiology 150 (Pt 7):2257-2266).

Das Gen, das für die $\beta$-Xylosidase XynB1 aus *G. stearothermophilus* codiert, ist Teil eines Genclusters, das für weitere am Abbau von Xylan beteiligte Enyzme codiert. So enthält dieser neben dem Gen für die $\beta$-Xylosidase XynB1

auch Gene für Xylartasen und $\alpha$-Glukuronidasen (Shulami et al., 1999, J. Bacteriol. 181(12):3695-3704). Auch die für die $\beta$-Xylosidasen aus *Thermo anaerobacter brockii* und *Thermoanaerobacterium* sp. JW/SL YS485 codierenden Gene sind direkt neben Genen lokalisiert, die für eine $\beta$-Glukosidase bzw. eine Acetylxylan-Esterase codieren (Breves et al., 1997, Appl. Environ. Microbiol. 63(10):3902-3910; Lorenz & Wiegel, 1997, J. Bacteriol. 179(17):5436-5441).

**Industrielle Anwendungen cellulolytischer und xylanolytischer Enzyme**

Der Einsatz von cellulolytischen und xylanolytischen Enzymen ist in der Industrie weit verbreitet. So finden sie Anwendung in der Nahrungs- und Futtermittelproduktion, aber auch in der Papier- und Zellstoff- sowie in der Textilindustrie werden Cellulasen und Xylanasen eingesetzt. Ihre Verwendung dient dabei der Erhöhung der Ausbeute und einer Verbesserung der Qualität. Des Weiteren erfolgt der Einsatz der Enzyme als umweltschonende Alternative zu herkömmlichen Behandlungsmethoden.

In der Nahrungsmittelindustrie werden cellulolytische und xylanolytische Enzyme zur Herstellung von Fruchtsäften, Wein und Bier, sowie bei der Extraktion von Öl, vorzugsweise Olivenöl, Rapsöl und Sonnenblumenöl und in der Backwarenindustrie verwendet. Bei der Herstellung von Fruchtsäften werden Cellulasen und Hemicellulasen gemeinsam mit Pektinasen zur Steigerung der Saftausbeute und Klärung von Fruchtsäften genutzt. Durch Behandlung des Fruchtfleischs mit den Enzymen wird die Saftbildung erhöht und gleichzeitig die Prozessdauer verkürzt. Durch die anschließende Klärung des Safts mit Hilfe der Enzyme wird die Viskosität verringert und somit die Filtrierbarkeit verbessert. Aber auch bei der Herstellung von Öl, vorzugsweise Olivenöl, Rapsöl und Sonnenblumenöl werden Cellulasen und Hemicellulasen zusammen mit Pektinasen für die Steigerung der Extraktion eingesetzt. Diese Enzyme finden ebenfalls bei der Herstellung von Weinen Anwendung, wo sie neben der Klärung auch zur Extraktion der in den Früchten vorhandenen Farbstoffe und zur Verbesserung des Weinaromas beitragen.

Außerdem werden Cellulasen in Brauereien zur Hydrolyse des Gerstenglukans eingesetzt, um die Filtrierbarkeit des Biers zu erhöhen. Xylanasen werden auch in der Backwarenindustrie eingesetzt. Dort tragen sie als Mehlzusatz zu einer besseren Verarbeitbarkeit des Teigs bei und führen zu einer verbesserten Qualität der Backwaren (Beg et al., 2001, Appl. Microbiol. Biotechnol. 56(3-4);326-338; Bhat, 2000, Biotechnol. Adv. 18(5):355-383; Galante et al., 1998, in: Enzymes, biological control and commercial applications, eds Harman GE & Kubicek CP, Verlag Taylor & Francis, London, Vol. 2, Seiten 327-342; Bhat & Bhat, 1997, Biotechnol. Adv. 15(3-4):583-620).

Cellulasen und Xylanasen finden auch in der Futtermittelindustrie Verwendung. Dabei tragen die Enzyme durch den Aufschluss der Cellulose und Hemicellulose in pflanzlichen Futtermitteln zu einer Erhöhung des Nährwerts und einer besseren Verdaubarkeit des Futters bei (Bhat & Bhat, 1997, Biotechnol. Adv. 15(3-4):583-620).

In der Papier- und Zellstoffindustrie werden Cellulasen und Xylanasen zur Modifikation der Holzfaserstruktur eingesetzt, um so eine bessere Weiterverarbeitung der Pulpe zu ermöglichen. Aber auch der Einsatz von Xylanasen bei der Papierbleiche ist weit verbreitet. Dabei führt die Behandlung der Pulpe mit Endoxylanasen zur Freisetzung von Lignin, wodurch die Zellwand der Holzfasern für Bleichmittel leichter zugänglich wird und so der Einsatz von Bleichmitteln deutlich verringert werden kann (Bhat, 2000, Biotechnol. Adv. 18(5):355-383; Buchert et al., 1998, in: Trichoderma & Gliocladium - Enzymes, biological control and commercial applications, eds Harman GE & Kubicek CP, Verlag Taylor & Francis, London, Vol 2, pp 343-363).

Beta-Pyranosidasen werden im Stand der Technik in der WO 2009/1416464 und der WO 2009/094187 beschrieben. Beispielhafte Sequenzen sind als "*T. neapolitana* beta-xylosidase polypeptide" unter accession no. GSP:AXV95559 XP002667899 und "*T. maritima* MSB8 beta-xylosidase protein" unter accession no. GSP:AXF64468 und XP002667901 hinterlegt. Das glycoside hydrolase family 3 domain protein" ist unter accession no. A8F429 und XP002667902 hinterlegt.

**OFFENBARUNG DER ERFINDUNG**

**Definitionen**

Der Begriff "Glykosid-Hydrolase" bezieht sich auf ein Protein mit einer enzymatischen Aktivität, das befähigt ist, glykosidische Bindungen zwischen Kohlenhydraten bzw. einem Kohlenhydrat und einem nicht Kohlenhydrat-haltigen Rest zu hydrolysieren.

Der Begriff "β-Pyranosidase" bezieht sich auf eine Glykosid-Hydrolase mit der Aktivität einer β-Glykosidase (E.C. 3.2.1.-), die befähigt ist, die Hydrolyse einer β-glykosidischen Bindung an einer Pyranose zu katalysieren.

Die Begriff "β-Glukopyranosidase" und "β-Glukosidase" beziehen sich auf eine Glykosid-Hydrolase, die befähigt ist, die Hydrolyse einer β-glykosidischen Bindung an einer Glukose zu katalysieren, unter Entstehen niedermolekularer Glukose-Oligomere oder -Monomere.

"Polypeptid" ist ein Oligo- oder Polymer aus Aminosäurebausteinen, die miteinander durch Peptidbindungen verknüpft sind.

Der Begriff "mono- oder oligomerer Baustein" schließt alle mono- oder oligomeren Bausteine ein, die durch enzymatische Aktivität aus einem Polymer gelöst werden können. Der Begriff Oligomer schließt alle Verbindungen aus mindestens zwei Bausteinen ein.

"Familie 3 Glykosid-Hydrolase" oder "Glykosid-Hydrolase der Familie 3" bezieht sich auf ein Polypeptid mit Glykosid-Hydrolase-Aktivität, das nach Henrissat et al. (1998, FEBS Lett. 425(2):352-354) der Familie 3 zugeordnet wird.

"Strukturdomäne 1" der Glykosid-Hydrolase 3 bezieht sich auf eine Domäne, die mindestens 50 %, bevorzugt mindestens 70 % und noch bevorzugter mindestens 90 % Identität der Aminosäuresequenz aufweist mit dem Sequenzabschnitt, der die Aminosäurereste 1 bis 357 der SEQ ID NO. 4 (Gersten-*β*-D-Glukan-Exohydrolase-Isoenzym Exo I, Genbank Referenznummer AF102868.1, veröffentlicht in: Varghese et al., 1999, Structure, Vol. 7, No. 2, p 179-190) umfasst.

"Strukturdomäne 2" der Glykosid-Hydrolase 3 bezieht sich auf eine Domäne, die mindestens 50 %, bevorzugt mindestens 70 % und noch bevorzugter mindestens 90 % Identität der Aminosäuresequenz aufweist mit dem Sequenzabschnitt, der die Aminosäurereste 374 bis 559 der SEQ ID. NO. 4 (Gersten-*β*-D-Glukan-Exohydrolase-Isoenzyms Exo I, Varghese et al., 1999, Structure, Vol. 7, No. 2, p 179-190) umfasst.

Der Begriff "% Identität" bezogen auf Aminosäure- oder Nucleotidsequenzen bezieht sich auf den Prozentwert, der ermittelt wird, wenn folgende Methode angewandt wird: Das Alignment zweier Aminosäuresequenzen miteinander oder zweier Nucleinsäure-Sequenzenen miteinander wird mit AlignX durchgeführt. AlignX ist eine Stand-Alone Anwendung, die von Invitrogen mit Vector NTI Advance 10.3.0 vertrieben wird. Der Algorithmus für die Alignments ist der ClustalW-Algorithmus (Nucleic Acid Research, 22 (22): 4673-4680, 1994). Als Parameter werden dabei für die Lückenöffnungsstrafe (Gap opening penalty) der Wert 15/10 und für die Lückenverlängerungsstrafe (Gap extension penalty) der Wert 6.66/0.1 verwendet.

"Ähnliche Sequenzen" sind charakterisiert durch bestimmte mindeste prozentuale Identitäten untereinander, ausgedrückt durch einen zahlenwert, zum Beispiel mindestens 30 %, mindestens 50 %, mindestens 70 % oder mindestens 90 %, sowie auf Sequenzen oder Sequenzabschnitte, deren gemeinsamer evolutionärer Ursprung durch andere Kriterien, wie z.B. Strukturvergleiche, nachgewiesen werden kann.

Der Begriff "Mutation" umfasst jegliche Art von Nucleotidesquenzmodifikation oder Aminosäuresequenzmodifikation, einschließlich Deletionen, Insertionen, Punktmutationen, Inversionen oder Kombinationen daraus.

"pNP" steht für para-Nitrophenyl.

"Thermostabilität" oder "Temperaturstabilität" bezieht sich auf eine Eigenschaft eines Enzyms, die ermittelt werden kann, indem eine Enzympräparation in zwei Fraktionen aufgeteilt wird, von denen eine einer bestimmten Temperatur ausgesetzt wird und anschließend die Aktivität dieser Fraktion nach der Inkubationsperiode bei der bestimmten Temperatur verglichen wird mit der Aktivität derjenigen Fraktion, die nicht bei der bestimmten Temperatur inkubiert wurde. Der entsprechende Wert wird typischerweise in % angegeben.

"pH-Stabilität" bezieht sich auf eine Eigenschaft eines Enzyms, die ermittelt werden kann, indem eine Enzympräparation in zwei Fraktionen aufgeteilt wird, von denen eine einem bestimmten pH-Wert ausgesetzt wird und anschließend die Aktivität dieser Fraktion nach der Inkubationsperiode bei dem bestimmten pH-Wert verglichen wird mit der Aktivität derjenigen Fraktion, das nicht bei dem bestimmten pH-Wert inkubiert wurde. Der entsprechende Wert wird typischerweise in % angegeben.

## Kurze Beschreibung der Erfindung

Die vorliegende Erfindung betrifft Polypeptide mit *β*-Pyranosidase-Aktivität die eine Aminosäuresequenz umfassen, die mindestens 75 %, möglichst mindestens 80 %, und vorzugsweise mindestens 85 % Sequenzidentität mit SEQ ID NO. 2 haben. SEQ ID NO. 2 ist in SEQ ID NO. 1 enthalten. SEQ ID NO.1 enthält ein Polypeptid, das im Folgenden *Fg*Xyl3a genannt wird. Die zugrunde liegende DNA wird dargestellt durch SEQ ID NO. 3. Sie wurde aus dem Genbank-Klon Bgl13 isoliert, der einen Abschnitt genomischer DNA aus *Fervidobacterium gondwanense* (*F. gondwanense*) enthält.

Die Erfindung betrifft auch Polypeptide, die eine Aminosäuresequenz umfassen, die mindestens 75 %, möglichst mindestens 80 %, und vorzugsweise mindestens 85 % Sequenzidentität mit SEQ ID NO. 1 haben.

Weiterhin offenbart die Anmeldung Polypeptide, die eine Strukturdomäne 1 einer Glykosid-Hydrolase der Familie 3 (GHF3) und eine Strukturdomäne 2 einer Glykosid-Hydrolase der Familie 3 (GHF3) umfassen, wobei mindestens eine dieser beiden Domänen mindestens 70 %, möglichst mindestens 75 %, möglichst mindestens 80 %, und vorzugsweise mindestens 85 % Sequenzidentität mit der der entsprechenden Domäne *Fg*Xyl3a, hat. Die Strukturdomäne 1 von *Fg*Xyl3a besteht dem Bereich der die Aminosäurereste 13 bis 381 sowie 586 bis 383 aus SEQ ID NO. 1 umfasst; die Strukturdomäne 2 von *F*gXyl3a besteht dem Bereich, der die Aminosäurereste 382 bis 585 aus SEQ ID NO. 1 umfasst.

Die *β*-Pyranosidase-Aktivität des Polypeptids im Sinne dieser Erfindung wird ausgewählt aus *β*-Xylopyranosidase-Aktivität, *β*-Glukopyranosidase-Aktivität und einer Kombination dieser beiden Aktivitäten, wobei die *β*-Xylopyranosidase-Aktivität bevorzugt ist.

In einer bevorzugten Ausführungsform betrifft die vorliegende Erfindung ein Polypeptid mit *β*-Pyranosidase-Aktivität, dessen Aminosäuresequenz mindestens einem der oben genannten Kriterien entspricht und das mindestens eine $\beta$-glykosidische Bindung spalten kann, die in den folgenden Substraten enthalten ist: Xylobiose, Xylotriose, Xylotetraose, Xylan.

In einer bevorzugteren Ausführungsform betrifft die vorliegende Erfindung ein Polypeptid mit $\beta$-Pyranosidase-Aktivität, das mindestens einem der oben genannten Kriterien entspricht, und dessen Aktivität gegenüber pNP-$\beta$-Cellobiosid deutlich geringer ist als gegenüber pNP-$\beta$-Xylobiosid, und zwar 5 % oder weniger gegenüber pNP-$\beta$-Cellobiosid, verglichen mit der Aktivität gegenüber pNP-$\beta$-Xylobiosid. (pNP steht in diesem Anmeldetext für para-Nitrophenyl).

In einer noch bevorzugteren Ausführungsform betrifft die vorliegende Erfindung ein Polypeptid mit $\beta$-Pyranosidase-Aktivität, das mindestens einem der oben genannten Kriterien entspricht, und dessen Aktivität gegenüber pNP-$\beta$-Xylopyranosid im sauren, neutralen oder leicht basischen Bereich, das heißt in einem Bereich von pH 4,0 bis pH 8,0, davon möglichst im leicht sauren bis neutralen Bereich, das heißt in einem Bereich von pH 5,5 bis 7,0, und vorzugsweise in einem Bereich von pH 6,2 bis 6,8 maximal ist.

In einer noch bevorzugteren Ausführungsform betrifft die vorliegende Erfindung ein pH-stabiles Polypeptid mit $\beta$-Pyranosidase-Aktivität, das mindestens einem der oben genannten Kriterien entspricht, und dessen maximale Aktivität gegenüber pNP-$\beta$-Xylopyranosid nach 48 Stunden Inkubation bei pH 9,0 mindestens 50 %, möglichst mindestens 60 %, möglichst mindestens 70 %, und vorzugsweise mindestens 80 % beträgt.

In einer noch bevorzugteren Ausführungsform betrifft die vorliegende Erfindung ein Hochtemperatur-aktives Polypeptid mit $\beta$-Pyranosidase-Aktivität, das mindestens einem der oben genannten Kriterien entspricht, und dessen Aktivität gegenüber pNP-$\beta$-Xylopyranosid in einem Bereich zwischen 60 °C und 100 °C, möglichst zwischen 70 °C und 95 °C, und vorzugsweise zwischen 80 °C und 90 °C maximal ist.

In einer noch bevorzugteren Ausführungsform betrifft die vorliegende Erfindung ein temperaturstabiles Polypeptid mit $\beta$-Pyranosidase-Aktivität, das mindestens einem der oben genannten Kriterien entspricht, und dessen maximale Aktivität gegenüber pNP-$\beta$-Xylopyranosid nach 3 Stunden Inkubation bei 60 °C mindestens 40 %, vorzugsweise mindestens 50 % beträgt.

In einer noch bevorzugteren Ausführungsform kann das Polypeptid, das mindestens einem der oben beschriebenen Kriterien entspricht, als Fusionsprotein vorkommen, wobei es bevorzugt mit einem der folgenden fusioniert ist: einer Kohlenhydrat-Bindedomäne eines anderen Proteins, einem Signalpeptid, einen Affinitäts-Tag oder einer Protease-Schnittstelle.

Die vorliegende Erfindung betrifft auch eine Mischung, die das Polypeptid mit $\beta$-Pyranosidase-Aktivität, das mindestens einem der oben genannten Kriterien entspricht, und eine oder mehrere Pektinasen und/oder eine oder mehrere Endoxylanasen und/oder eine oder mehrere $\beta$-Glukosidasen und/oder eine oder mehrere $\beta$-Glukanasen und/oder eine oder mehrere Cellobiohydrolasen und/oder eine oder mehrere $\beta$-Xylosidasen und/oder eine oder mehrere $\alpha$-Arabinofuranosidasen und/oder eine oder mehrere $\alpha$-Glukuronidasen und/oder eine oder mehrere Acetylxylan-Esterasen enthält.

Die vorliegende Erfindung betrifft auch eine Nucleinsäure, die für das oben beschrieben Polypeptid codiert, sowie einen Vektor, der diese Nucleinsäure enthält.

Die vorliegende Erfindung betrifft auch eine Wirtszelle, die mit dem oben beschriebenen Vektor transformiert wurde. Die Wirtszelle im Sinne dieser Erfindung kann ein Prokaryont oder ein Eukaryont sein. Eine eukaryontische Wirtszelle wird bevorzugt ausgewählt aus der Gruppe bestehend aus *Saccharomyces cerevisiae, Yarrowia lipolytica, Schizosaccharomyces lactis, Klyuveromyces lactis, Pichia methanolytica*, *Pichia pastoris*, *Pichia angusta*, *Hansenula polymorpha*, *Aspergillus niger, Chrysosporium lucknowense*, *Trichoderma reesei*, *Penicillum* sp.

In einer besonders bevorzugteren Ausführungsform ist die eukaryontische Wirtszelle eine methylotrophische Hefe, vorzugsweise aus der Gruppe, die *Pichia methanolytica*, *Pichia pastoria*, *Pichia angusta*, *Hansenula polymorpha* einschließt.

Eine prokaryontische Wirtszelle im Sinne dieser Erfindung wird bevorzugt ausgewählt aus der Gruppe, die *Bacillus* sp., *Bacillus subtilis*, *Bacillus licheniformis*, *Bacillus megaterium*, *Thermos thermophiuls, Pseudomonas fluorescence*, *Fervidobacterium* sp., *Escherichia coli* umfasst.

Die vorliegende Erfindung betrifft auch ein Verfahren zur Aufreinigung des oben beschriebenen Polypeptids mit $\beta$-Pyranosidase-Aktivität, das folgende Schritte umfasst:

a) Gewinnen der Wirtszelle, die mit dem oben beschriebenen Vektor transformiert wurde
b) Aufzucht der Wirtszelle unter Bedingungen unter denen das Polypeptid mit $\beta$-Pyranosidase-Aktivität exprimiert wird
c) Aufreinigung des Polypeptids mit $\beta$-Pyranosidase-Aktivität.

In einer bevorzugten Ausführungsform enthält Schritt c) des Verfahrens zur Aufreinigung des oben beschriebenen Polypeptids mit $\beta$-Pyranosidase-Aktivität eine Hitzefällung.

Die vorliegende Erfindung betrifft auch die Verwendung des oben beschriebenen Polypeptids mit $\beta$-Pyranosi-

dase-Aktivität oder der oben beschriebenen Mischung die das oben beschriebenen Polypeptids mit β-Pyranosidase-Aktivität enthält, zum Abbau von einem oder mehreren der folgenden Substrate: β-Xylopyranosid oder β-Glukopyranosid.

In einer bevorzugten Ausführungsform betrifft die vorliegende Erfindung die oben beschriebene Verwendung für den enzymatischen Abbau von lignocellulosehaltiger Biomasse; und/oder für die Textilverarbeitung, und/oder als Zusatz von Detergenzien und/oder in der Lebensmittel- und/oder Futterindustrie.

In einer bevorzugten Ausführungsform betrifft die vorliegende Erfindung die oben beschriebene Verwendung für die Herstellung von Fruchtsäften, und/oder für die Herstellung von Wein oder weinhaltigen Getränken, und/oder für die Herstellung von Bier oder bierhaltigen Getränken, und/oder für die Herstellung von Öl, vorzugsweise für die Herstellung von Olivenöl, Rapsöl oder Sonnenblumenöl und/oder für die Herstellung von Backwaren.

## Detaillierte Beschreibung der Erfindung

### Polypeptid mit β-Pyranosidase-Aktivität

Die vorliegende Erfindung betrifft Polypeptide mit β-Pyranosidase-Aktivität, die eine Aminosäuresequenz umfassen, die mindestens 71 %, möglichst mindestens 75 %, möglichst mindestens 80 %, und vorzugsweise mindestens 85 % Sequenzidentität mit SEQ ID NO. 2 haben. Die Sequenzidentität wird dabei ermittelt, wie im Abschnitt "Definitionen" beschrieben.

SEQ ID NO. 2 ist in SEQ ID 1 enthalten. SEQ ID NO. 1 enthält die Aminosäuresequenz des Polypeptids *Fg*Xyl3a. Die der SEQ ID NO. 1 zugrundeliegende DNA, dargestellt durch SEQ ID NO. 3, stammt aus dem Genbank-Klon Bgl13, der einen Abschnitt genomischer DNA aus *Fervidobacterium gondwanense* (*F. gondwanense*), enthält. Das Auffinden der SEQ ID NO. 3 sowie die Ermittlung der Domänenstruktur sind in Beispiel 3 detailliert beschrieben.

Kurz, SEQ ID NO. 2 umfasst denjenigen Bereich von SEQ ID NO. 1, der beide Strukturdomänen der Glykosidhydrolase enthält durch einen Sequenzvergleich mit SEQ ID NO.4 (Enzym BglB aus *Thermotoga neapolitana,* Pozzo et al., 2010, J. Mol. Biol., 2:397(3), 724-739) ermittelt wurde. SEQ ID NO. 2 umfasst also die Aminosäurereste 13 bis 638 der SEQ ID NO. 1.

Die vorliegende Erfindung betrifft auch Polypeptide, die eine Aminosäuresequenz umfassen, die mindestens 75 %, möglichst mindestens 80 %, und vorzugsweise mindestens 85 % Sequenzidentität mit SEQ ID NO. 1 haben.

Weiterhin offenbart die Anmeldung Polypeptide, die eine Strukturdomäne 1 einer Glykosid-Hydrolase der Familie 3 (GHF3) und eine Strukturdomäne 2 einer Glykosid-Hydrolase der Familie 3 (GHF3) umfassen, wobei mindestens eine dieser beiden Domänen mindestens 70 %, möglichst mindestens 75 %, möglichst mindestens 80 %, und vorzugsweise mindestens 85 % Sequenzidentität mit der der entsprechenden Domäne *Fg*Xyl3a, hat. Die Strukturdomäne 1 von *Fg*Xyl3a besteht aus dem Bereich der die Aminosäurereste 13 bis 381 sowie 586 bis 383 aus SEQ ID NO. 1 umfasst; die Strukturdomäne 2 von *Fg*Xyl3a besteht aus dem Bereich der die Aminosäurereste 382 bis 585 aus SEQ ID NO. 1 umfasst. Die Methode zur Ermittlung der Strukturdomänen ist in Beispiel 1 beschrieben. Sie kann allgemein auf Polypeptide angewendet werden, deren Sequenzidentität mindestens den hier beschriebenen Kriterien entspricht.

Die Klonierung (Beispiel 2), Expression (Beispiel 3), Reinigung aus einem Zellextrakt (Beispiele 4 und 5) sowie die Ermittlung des Molekulargewichts (Beispiel 6) eines Polypeptids mit β-Pyranosidase-Aktivität im Sinne dieser Erfindung ist in den Beispielen beschrieben.

Die β-Pyranosidase-Aktivität des Polypeptids im Sinne dieser Erfindung wird ausgewählt aus β-Xylopyranosidase-Aktivität, β-Glukopyranosidase-Aktivität und einer Kombination dieser beiden Aktivitäten, wobei die β-Xylopyranosidase-Aktivität bevorzugt ist. Aktivitätsnachweis sowie Ermittlung der Substratspezifität eines Polypeptids im Sinne dieser Erfindung wird in den Beispielen 7 und 8 ausführlich beschrieben.

In einer bevorzugten Ausführungsform betrifft die vorliegende Erfindung ein Polypeptid mit β-Pyranosidase-Aktivität, das mindestens einem der oben genannten Kriterien entspricht, und das mindestens eine der in den folgenden Substraten enthaltenen β-glykosidische Bindungen hydrolysieren kann: Xylobiose, Xylotriose, Xylotetraose, Xylan. Die Ermittlung der Hydrolyseprodukte eines Polypeptids mit β-Pyranosidase-Aktivität ist in Beispiel 13 detailliert beschrieben.

In bevorzugteren Ausführungsform betrifft die vorliegende Erfindung ein Polypeptid mit β-Pyranosidase-Aktivität, das mindestens einem der oben genannten Kriterien entspricht, und dessen Aktivität gegenüber pNP-β-Cellobiosid deutlich geringer ist als gegenüber pNP-β-Xylobiosid, und zwar 5 % oder weniger gegenüber pNP-β-Cellobiosid, verglichen mit der Aktivität gegenüber pNP-β-Xylobiosid. Ein Verfahren zur Ermittlung der Substratspezifität ist in Beispiel 8 beschrieben.

In einer noch bevorzugteren Ausführungsform betrifft die vorliegende Erfindung ein Polypeptid mit β-Pyranosidase-Aktivität, das mindestens einem der oben genannten Kriterien entspricht, und dessen Aktivität gegenüber pNP-β-Xylopyranosid im sauren, neutralen oder leicht basischen Bereich, das heißt in einem Bereich von pH 4,0 bis pH 8,0, davon möglichst im leicht sauren bis neutralen Bereich, das heißt in einem Bereich von pH 5,5 bis 7,0, und vorzugsweise in einem Bereich von pH 6,2 bis 6,8 maximal ist. Ein Verfahren zur Ermittlung des Einflusses des pH-Werts auf die Aktivität eines Polypeptids mit β-Pyranosidase-Aktivität ist in Beispiel 11 genauer beschrieben.

In einer noch bevorzugteren Ausführungsform betrifft die vorliegende Erfindung ein pH-stabiles Polypeptid mit $\beta$-Pyranosidase-Aktivität, das mindestens einem der oben genannten Kriterien entspricht, und dessen maximale Aktivität gegenüber pNP-$\beta$-Xylopyranosid nach 48 Stunden Inkubation bei pH 9,0 mindestens 50 %, möglichst mindestens 60 %, möglichst mindestens 70 %, und vorzugsweise mindestens 80 % beträgt. Ein Verfahren zur Ermittlung des Einflusses des pH-Werts auf die Aktivität eines Polypeptids mit $\beta$-Pyranosidase-Aktivität ist in Beispiel 11 genauer beschrieben.

In einer noch bevorzugteren Ausführungsform betrifft die vorliegende Erfindung ein Hochtemperatur-aktives Polypeptid mit $\beta$-Pyranosidase-Aktivität, das mindestens einem der oben genannten Kriterien entspricht, und dessen Aktivität gegenüber pNP-$\beta$-Xylopyranosid in einem Bereich zwischen 60 °C und 100 °C, möglichst zwischen 70 °C und 95 °C, und vorzugsweise zwischen 80 °C und 90 °C maximal ist. Ein Verfahren zur Ermittlung des Einflusses der Temperatur auf die Aktivität eines Polypeptids mit $\beta$-Pyranosidase-Aktivität ist in Beispiel 9 genauer beschrieben.

In einer noch bevorzugteren Ausführungsform betrifft die vorliegende Erfindung ein temperaturstabiles Polypeptid mit $\beta$-Pyranosidase-Aktivität, das mindestens einem der oben genannten Kriterien entspricht, und dessen maximale Aktivität gegenüber pNP-$\beta$-Xylopyranosid nach 3 Stunden Inkubation bei 60 °C mindestens 40 %, vorzugsweise mindestens 50 % beträgt. Ein Verfahren zur Ermittlung der Temperaturstabilität auf die Aktivität eines Polypeptids mit $\beta$-Pyranosidase-Aktivität ist in Beispiel 10 genauer beschrieben.

**Fusionsprotein**

Das Polypeptid im Sinne dieser Erfindung kann entweder isoliert auftreten, oder mit ein oder mehreren weiteren Oligo- oder Polypeptiden fusioniert sein. Das heißt, in einer weiteren bevorzugten Ausführungsform kann das Polypeptid, das mindestens eines der oben beschriebenen Kriterien erfüllt, als Fusionsprotein vorkommen, wobei es bevorzugt mit einem der folgenden fusioniert ist: einer Kohlenhydrat-Bindedomäne eines anderen Proteins, einem Signalpeptid, einen Affinitäts-Tag oder einer Protease-Schnittstelle.

Das Fusionsprotein im Sinne dieser Erfindung ist nicht auf die Methode beschränkt, mit der es gewonnen wurde, sondern umfasst Fusionsproteine aller Art, sofern darin ein Bestandteil enthalten ist, der mindestens eines der oben genannten Kriterien erfüllt. Eine Möglichkeit ist, das Fusionsprotein im Sinne dieser Erfindung mit Hilfe molekularbiologischer Methoden zu gewinnen. Wie der Fachmann weiß, sind Verfahren zum Erstellen von Nukleinsäuren, die für Fusionsproteine codieren, molekularbiologische Standardmethoden, die zum Beispiel in Sambrook *et al*. (Molecular cloning, a laboratory manual, 2nd edition, 1989, Cold Spring Harbor Laboratory Press, Cold Spring Harbor, New York) genauer beschrieben sind. Dabei kann das Fusionsprotein neben dem Polypeptid mit $\beta$-Xylosidase-Aktivität im Sinne dieser Erfindung in einer bevorzugten Ausführungsform fusioniert werden mit einer Kohlenhydrat-Bindedomäne eines anderen Proteins. In einer bevorzugten Ausführungsform kann das Polypeptid mit $\beta$-Xylosidase-Aktivität im Sinne dieser Erfindung fusioniert sein mit einem Polypeptid, das ausgewählt wird aus einem oder mehreren der aus der folgenden Gruppe: Signalpeptid, Affinitäts-Tag, Protease-Schnittstelle. Eine translationelle Kupplung kann dabei benützt werden, um das exprimierte Polypeptid im Sinne der vorliegenden Erfindung zu zellulären Kompartimenten, Organellen oder zum Export aus der Wirtszelle zu dirigieren. Signalpeptide, auch genannt Signalsequenzen, sind dem Fachmann wohl bekannt, und schließen die Leitsequenzen der periplasmatischen Proteine OmpA, *OmpT, Pel*B, *Pho*A, ein. Signalsequenzen für den Export von Proteinen kommen zum Beispiel in natürlich vorkommenden sekretierten Proteinen vor, zum Beispiel Proteinen mit Kohlenhydrat-modifizierenden Eigenschaften, wie Cellobiohydrolase I oder II, Endoglucanasae, *Amy*E, sowie in *S. cerevisiae* Mf$\alpha$ oder Hühnerei-Lysozym. Schnittstellen für Proteasen, die sich als Anhängsel rekombinant exprimierter Proteine eignen, sind dem Fachmann wohl bekannt. Protease-Schnittstelle bedeutet dabei ein Pofy- oder Oligopeptid, das eine Peptidbindung umfasst, die von einer bestimmten Protease spezifisch gespalten werden kann, sowie eine Erkennungssequenz, die sich in der Regel in der Nähe der Spaltstelle befindet, und die von der entsprechenden Protease erkannt wird. Die Protease-Schnittstellen die im Sinne dieser Erfindung verwendet werden können, sind nicht weiter limitiert. Sie schließen ausdrücklich die Schnittstelle der *tobacco etch virus* (TEV) Protease, die Schnittstellen der Blutgerinnungsfaktoren aus Säugetieren, wie zum Beispiel Faktor Xa oder Thrombin ein. Die Affinitäts-Tags, die im Sinne dieser Erfindung verwendet werden können, sind nicht weiter limitiert. Wie der Fachmann weiß, sind Affinitäts-Tags, die sich für die Reinigung von vielen Polypeptiden als vorteilhaft erwiesen haben, zum Beispiel in Sambrook et al. (Molecular cloning, a laboratory manual, 2nd edition, 1989, Cold Spring Harbor Laboratory Press, Cold Spring Harbor, New York) genauer beschrieben. Ausdrücklich eingeschlossen im Sinne dieser Erfindung ist ein Oligo-Histidin-Tag, wie in den Beispielen 1 und 5 beschrieben.

Enzymmischung

Im Sinne der vorliegenden Erfindung kann auch eine Mischung von Enzymen verwendet werden. Diese enthält das Polypeptid mit $\beta$-Pyranosidase-Aktivität das mindestens einem der oben beschriebenen Kriterien umfasst, sowie ein, zwei oder mehr oder mehrere weitere Enzyme. Diese weiteren Enzyme können ausgewählt werden aus der Gruppe, die Pektinasen, Endoxylanasen, $\beta$-Glukosidasen, $\beta$-Glukanasen Cellobiohydrolasen, $\beta$-Xylosidasen, $\alpha$-Arabinofurano-

sidasen, $\alpha$-Glukuronidasen, Acetylxylan-Esterasen umfasst. Die Enzymmischung im Sinn der vorliegenden Erfindung umfasst also das Polypeptid mit $\beta$-Pyranosidase-Aktivität, das mindestens einem der oben beschriebenen Kriterien enthält, sowie eine oder mehrere Pektinasen und/oder eine oder mehrere Endoxylanasen und/oder eine oder mehrere $\beta$-Glukosidasen und/oder eine oder mehrere $\beta$-Glukanasen und/oder eine oder mehrere Cellobiohydrolasen und/oder eine oder mehrere $\beta$-Xylosidasen und/oder eine oder mehrere $\alpha$-Arabinofuranosidasen und/oder eine oder mehrere $\alpha$-Glukuronidasen und/oder eine oder mehrere Acetylxylan-Esterasen.

In einer bevorzugten Ausführungsform umfasst die Enzymmischung im Sinne der vorliegenden Erfindung das Polypeptid mit $\beta$-Pyranosidase-Aktivität, das mindestens einem der oben beschriebenen Kriterien enthält, sowie eine oder mehrere $\alpha$-Arabinofuranosidasen und/oder eine oder mehrere $\alpha$-Glurkuronidasen und/oder eine oder mehrere Acetylxylan-Esterasen.

Sofern nicht explizit anderweitig angegeben, wird der Begriff "umfasst" in dieser Anmeldung verwendet um anzugeben, dass weitere Komponenten optional zusätzlich zu den unter "umfasst" aufgeführten Komponenten anwesend sein können. Es wird aber als besondere Ausführungsform betrachtet dass der Begriff "umfasst" die Möglichkeit einschließt, dass keine weiteren Komponenten anwesend sind d.h. im Sinne dieser besonderen Ausführungsform wird der Begriff "umfasst" verstanden wie der Begriff "besteht aus".

### Nucleinsäure und Vektor

Die vorliegende Erfindung bezieht sich auch auf eine Nucleinsäure, die für das oben beschrieben Polypeptid codiert. Ein Beispiel für solch eine Nucleinsäure ist durch SEQ ID NO. 3 wiedergegeben. Die Nucleinsäure im Sinne dieser Erfindung kann Bestandteil einer Expressionskassette sein. Dem Fachmann sind die typischen Bestandteile einer Expressionskassette wohl bekannt; sie sind zum Beispiel in Sambrook et al. (Molecular cloning, a laboratory manual, 2nd edition, 1989, Cold Spring Harbor Laboratory Press, Cold Spring Harbor, New York) genauer beschrieben. Neben der codierenden Sequenz sind in der Expressionskassette typischerweise mindestens enthalten: ein Promotor und ein Terminator. Die Expressionslevel eines Gens, das für ein Polypeptid im Sinne dieser Erfindung codiert, können dabei angepasst werden durch die Kopienzahl des Gens, das in die Wirtszelle eingeführt wird, wobei vorzugsweise mehr als eine einzige Kopie vorhanden ist. Für optimierte Expression kann der Promotor angepasst werden, so dass er zum Beispiel auf die Zugabe einer Chemikalie oder auf Änderung eines oder mehrerer physikalischer Parameter dahingehend reagiert, das die Induktion des Gens induziert oder abgeschaltet werden kann. Beispiele für induzierbare Promotoren schließen das Tetracyclinrepressorsystem, das Lac-Repressorsystem (Beneyx, 1999, Curr. Opin. Biotechnol. 10:411:422), Kupferionen-induzierbare Systeme (Hottinger et al., 2004, Yeast, 10:283-296), methanolindzierbare Systeme (Cereghino et al., 2000, FEMS Microbiol. Reviews 24:45-66) oder den temperaturinduzierbaren $\lambda$PL-Promotor ein. Alternativ kann Derepression des Promotors durch Erreichen eines günstigen physiologischen Zustands der Kultur eine nützliche Strategie sein (Promotoren PhoA, Trp, Adh2, Fmdh, CBH1 (Price et al., 1990, Methods Enzymol., 185-308-318; Hollenberg, 1995, US3589585). Verfahren, um die Ausbeute noch weiter zu steigern, schließen Co-Expression eines oder mehrerer an der Translation beteiligten Proteine, an der Zielsteuerung beteiligten Proteine, an der Faltung beteiligten Proteine (zum Beispiel Chaperone der Hsp70-Familie, Protein-Disulfid-Isomerasen), oder von Proteasen, die bei der korrekten Prozessierung helfen, ein.

Die Expressionskassette kann dabei in einen Vektor integriert sein, der entweder episomal in der Wirtszelle propagiert wird, oder in dessen Genom integriert wird. Beispiele für typische Vektoren sind bakterielle Plasmide, Hefeplasmide, centromer-enthaltende lineare DNA, Konstrukte viralen Ursprungs wie SV40, Phagen-DNA, Bacculovirus, Vaccinia, Adenovirus, Geflügelpockenvirus, Schein-Tollwut, sowie Kombinationen von Vektoren bakteriellen, eukaryontischen und viralen Ursprungs. Integration kann erfolgen durch Methoden, die dem Fachmann der Molekularbiologie wohl bekannt sind, wie zum Beispiel homologe Rekombination, Transposition, oder durch Anwendung viraler Transfektionssysteme. Weiterhin sind episomale Systeme zur Expression, von denen sich ein oder mehrere Kopien geplant oder ungeplant in das Genom der Wirtszelle integrieren, eingeschlossen. Weiterhin sind alle Vektorsysteme eingeschlossen, die die heterologe Expression eines Polypeptids im Sinne der vorliegenden Erfindung in einer Wirtszelle ermöglichen.

### Wirtszelle

Die vorliegende Erfindung betrifft auch eine Wirtszelle, die mit dem oben beschriebenen Vektor transformiert wurde.

Bevorzugte Methoden für das Einführen des Vektorkonstrukts in die Wirtszelle schließen Transformation, Transfektion, Konjugation und Kreuzung ein. Die Transformation kann durch Elektroporation, Protoplastenfusion, Lipofektion, ballistische Bombardierung, chemische Transformation basierend auf Calciumchlorid, Polyethylenglycol (PEG) oder Manganchlorid erfolgen. Weitere Strategien schließen die Anwendung viraler Partikel ein. Eine weitere Möglichkeit ist die Verwendung natürlicherweise kompetenter Organismen als Wirtszellen.

Die Wirtszelle im Sinne dieser Erfindung kann ein Prokaryont oder ein Eukaryont sein. Eine eukaryontische Wirtszelle wird bevorzugt ausgewählt aus der Gruppe bestehend aus *Saccharomyces cerevisiae*, *Yarrowia lipolytica*, *Schizosaccharomyces lactis*, *Klyuveromyces lactis, Pichia methanolytica*, *Pichia pastoris*, *Pichia angusta, Hansenula polymorpha*, *Aspergillus niger, Chrysosporium lucknowense, Trichoderma reesei, Penicillum* sp..

In einer besonders bevorzugteren Ausführungsform ist die eukaryontische Wirtszelle eine methylotrophische Hefe, vorzugsweise aus der Gruppe, die *Pichia methanolytica*, *Pichia pastoria, Pichia angusta*, *Hansenula polymorpha* einschließt.

Eine prokaryontische Wirtszelle im Sinne dieser Erfindung wird bevorzugt ausgewählt aus der Gruppe, die *Bacillus* sp., *Bacillus subtitis*, *Bacillus licheniformis*, *Bacillus megaterium*, *Thermos thermophiuls, Pseudomonas fluorescence, Fervidobacterium* sp., *Escherichia coli* umfasst.

Nach der Transformation der Wirtszelle und Wachstum bis zur gewünschten Zelldichte kann der ausgewählte induzierbare Promotor induziert werden durch eines chemischen Aktivators oder durch Veränderung eines oder mehrer physikalischer Parameter, damit die kultivierten Wirtszellen das gewünschte Polypeptid produzieren.

**Verfahren zur Aufreinigung**

Die vorliegende Erfindung betrifft auch ein Verfahren zur Aufreinigung des oben beschriebenen Polypeptids mit $\beta$-Pyranosidase-Aktivität. In einer speziellen Ausführungsform, wird das Polypeptid dabei nicht von der Wirtszelle sekretiert. Im Anschluss an die Kultur werden die Wirtszellen dann isoliert, und das darin enthaltene Polypeptid im Sinne dieser Erfindung wird isoliert. Ein entsprechendes Verfahren ist in den Beispielen 3 bis 5 beschrieben. In einer besonderen Ausführungsform wird das Polypeptid im Sinne der vorliegenden Erfindung als Fusionsprotein mit mindestens einem Signalpeptid produziert, das das rekombinante Protein zur Sekretion aus der Wirtszelle dirigiert.

Im Einzelnen umfasst das Verfahren die folgenden Schritte:

a) Gewinnen der Wirtszelle, die mit dem oben beschriebenen Vektor transformiert wurde
b) Aufzucht der Wirtszelle unter Bedingungen unter denen das Polypeptid mit $\beta$-Pyranosidase-Aktivität exprimiert wird
c) Aufreinigung des Polypeptids mit $\beta$-Pyranosidase-Aktivität.

Die Wirtszelle nach a) kann nach Methoden gewonnen, die dem Fachmann wohl bekannt sind, z.B. wie im Kapitel "Wirtszelle" beschrieben.

Die Durchführungsform von Schritt b) wird von vielen Faktoren beeinflusst, die dem Fachmann grundsätzlich bekannt sind. So werden das Wachstumsmedium, Wachstumstemperatur und weitere Wachstumsbedingungen gewählt nach der Art der Wirtszelle, die verwendet wird. Die Bedingungen, unter denen das Polypeptid exprimiert wird, hängen darüber hinaus insbesondere von der Wahl des verwendeten Promotors ab, wie oben beschrieben. Verschiedene Verfahren zum Ernten von Wirtszellen aus einer proteinexprimierenden Zellkultur sind dem Fachmann wohl bekannt, und schließen z.B. Zentrifugation des Mediums, das die Wirtszellen enthält sowie Filtration des Mediums, das die Wirtszellen enthält, ein. Typischerweise werden die Zellen im Anschluss lysiert, entweder durch chemische oder mechanische Methoden, oder eine Kombination daraus.

Die Durchführungsform von Schritt c) wird gewählt in Abhängigkeit der Natur des exprimierten Polypeptids. In speziellen Ausführungsformen, in denen ein Fusionsprotein exprimiert wird, das das Polypeptid im Sinne dieser Erfindung und eine weitere Domäne, wie z.B. einen Affinitätstag besitzt, können auch die Eigenschaften des Affinitätstags ausgenutzt werden, um das Fusionsprotein aufzureinigen. Ein Beispiel für einen solchen Affinitätstag ist ein Anhang, der mehrere, typischerweise mindestens sechs, Histidinreste umfasst, wie in den beispielen 3 bis 5 beschrieben.

In einer bevorzugten Ausführungsform enthält Schritt c) des Verfahrens zur Aufreinigung des oben beschriebenen Polypeptids eine Hitzefällung. Hierzu wird eine Mischung verschiedener Polypeptide, die das gewünschte Polypeptid umfasst, auf eine bestimmte Temperatur gebracht, die in der Regel höher ist als die Temperatur, bei der die Wirtszelle während der Expression gewachsen ist. Diese Mischung kann zwei oder mehr unterschiedliche Polypeptide umfassen. Eine entsprechende Mischung kann auch das Lysat sein, das entsteht, wenn die Zellen, die das gewünschte Polypeptid exprimieren, lysiert werden, z.B. mit mechanischen oder chemischen Methoden oder einer Kombination daraus, oder ein aus diesem Lysat gewonnener oder angereicherter Extrakt.

Entsprechend ihren Eigenschaften fallen unterschiedliche Proteine bei unterschiedlichen erhöhten Temperaturen aus. So kann eine Anreicherung im Zell-Lysat oder dem daraus gewonnenen Extrakt erreicht werden. Durch weitere Temperatur-Erhöhung kann optional das gewünschte Protein ausgefällt werden.

**Verwendung**

Die vorliegende Erfindung betrifft auch die Verwendung des oben beschriebenen Polypeptids mit $\beta$-Pyranosidase-Aktivität oder der Mischung die das Polypeptids mit $\beta$-Pyranosidase-Aktivität enthält, zu einem beliebigen Zweck, zu dem solche Enzymaktivität benötigt oder gewünscht wird.

Die vorliegende Erfindung beschreibt insbesondere die Verwendung des oben beschriebenen Polypeptids mit *β*-Pyranosidase-Aktivität oder der Mischung die das Polypeptids mit *β*-Pyranosidase-Aktivität enthält, zum Abbau von einem oder mehreren Substraten, die ein oder mehrere *β*-xylopyranosidische und/oder ein oder mehrere *β*-glukopyranosidische Bindungen enthalten, im folgenden genannt *β*-Xylopyranosid oder *β*-Glukopyranosid.

In einer bevorzugten Ausführungsform bezieht sich die vorliegende Erfindung die oben beschriebene Verwendung für den enzymatischen Abbau von lignocellulosehaltiger Biomasse.

In einer bevorzugteren Ausführungsform bezieht sich die vorliegende Erfindung auf die oben beschriebene Verwendung in der Textilverarbeitung. In einer besonders bevorzugten Ausführungsform bezieht sich die vorliegende Erfindung auf die oben beschriebene Verwendung als Zusatz von Detergenzien. In einer weiteren besonders bevorzugten Ausführungsform bezieht sich die vorliegende Erfindung auf die oben beschriebene Verwendung in der Lebensmittel- und/oder Futterindustrie. In einer weiteren besonders bevorzugten Ausführungsform bezieht sich die vorliegende Erfindung schließlich auf eine Kombination von zwei oder mehr dieser Anwendungen.

In einer besonders bevorzugten Ausführungsform bezieht sich die vorliegende Erfindung auf die oben beschriebene Verwendung für die Herstellung von Fruchtsäften. In einer noch bevorzugteren Ausführungsform bezieht sich die vorliegende Erfindung auf die oben beschriebene Verwendung für die Herstellung von Wein oder weinhaltigen Getränken, und in einer weiteren noch bevorzugteren Ausführungsform bezieht sich die vorliegende Erfindung auf die oben beschriebene Verwendung für die Herstellung von Bier oder bierhaltigen Getränken. In einer weiteren noch bevorzugteren Ausführungsform bezieht sich die vorliegende Erfindung auf die oben beschriebene Verwendung für die Herstellung von Öl, vorzugsweise Olivenöl, Rapsöl oder Sonnenblumenöl. Schließlich bezieht sich die vorliegende Erfindung in einer weiteren noch bevorzugteren Ausführungsform auf die oben beschriebene Verwendung für die Herstellung von Backwaren.

## Beispiele

### Beispiel 1: Identifizierung und Charakterisierung der *β*-Pyranosidase *Fg*Xyl3A aus *Fervidobacterium gondwanense*

Zur Identifizierung von cellulolytischer und xylanolytischer Aktivität wurde eine Genbank von *F. gondwanense* gescreent. Die Genbank wurde nach folgender Methode hergestellt: Genomische DNA wurde aus *Fervidobacterium gondwanense* Zellen unter Verwendung des Qiagen's genomic DNA isolation kit (Qiagen GmbH, Hilden) gereinigt. Die gewonnene DNA wurde durch partiellen Verdau mit *Sau*3AI in klonierbare Fragmente von mehreren Kilobasen Länge abgebaut und diese mit lambda-ZAP-Express-Predigested (Stratagene™) Vektorarmen ligiert und anschließend, der Vorschrift des Herstellers folgend, die Ligationsprodukte in Phagenpartikel verpackt. Die Primärphagenbibliothek wurde in *E. coli* XL1-Blue MRF' Zellen amplifiziert und schließlich die Phagmid-Bibliothek mittels Helfer-Phagen ExAssist ausgeschnitten und nach Transfektion in *E. coli* XLOLR stabil etabliert.

Für die Durchmusterung wurden Bakterien des *E. coli*-Stamms XLOLR, die Teile des Genoms von *F. gondwanense* in dem Vektor pBK-CMV (Kanamycin-Resistenz, Phagemidvektor mit Lac-Promotor, Agilent Technologies, Waldbronn) enthielten, auf LB-Selektionsböden ausplattiert und über Nacht bei 37°C inkubiert. Die gewachsenen Kolonien wurden auf frische Nährböden übertragen und erneut bei 37°C über Nacht inkubiert.

Um Kolonien mit *β*-Glukosidase-Aktivität zu identifizieren, wurde Esculin (Sigma-Aldrich, München) als Substrat eingesetzt. Hierzu wurden die Kolonien mit Esculin-Agar überschichtet (0,1% (w/v) Esculin, 0,01% (w/v) Ammoniumeisen(III)-Citrat, 50 mM Na-Acetat, 1% (w/v) Agarose, pH 6,0) nachgewiesen. Dabei erfolgte der Aktivitätsnachweis durch Bildung eines braunen Hofs um die Kolonien, der auf der Spaltung des Esculins zu Glukose und Esculetin beruht. Esculetin bildet mit Eisenionen einen Komplex, der durch eine Braunfärbung sichtbar wird. Enzymatisch aktive Klone wurden isoliert und kultiviert.

Das Screening mit dem Substrat Esculin mit Hilfe kolorimetrischer Nachweisverfahren führte zu der Identifizierung des Genbank-Klons Bgl13.

Zur Ermittlung des für das aktive Protein codierenden offenen Leserahmen (open reading frames, ORFs) wurde das Plasmid aus Klon Bugl13 mittels "Primer Walking" unter Verwendung der Standardprimer T3 und T7 sequenziert.

| | |
|---|---|
| T3 | 5'-ATTAACCCTCACTAAAGGGA-3' |
| T7 | 5'-TAATAGGACTGACTATAGGG-3' |

Diese Standardmethode ist in Sambrook et al. (Molecular cloning, a laboratory manual, 2nd edition, 1989, Cold Spring Harbor Laboratory Press, Cold Spring Harbor, New York) genauer beschrieben.

Die Gesamtgröße des Inserts betrug 8479 Basenpaare (bp). Zur Identifizierung von offenen Leserahmen auf dem Insert wurde der "ORF Finder" der NCBI-Datenbank verwendet, wobei fünf offene Leserahmen bestimmt wurden. Um den Proteinen, die von den ORFs codiert werden, eine mögliche Funktion zuordnen zu können, wurden ihre Aminosäuresequenzen mittels "blastp" der NCBI-Datenbank mit bekannten Sequenzen verglichen. BLAST steht für "Basic Local Alignment Search Tool" (Altschul et al., 1997, Nucleic Acids Res 25(17):3389-3402). Die Aminosäuresequenz von ORF 1 zeigte dabei Ähnlichkeiten zu $\beta$-Xylosidasen der Glykosid-Hydrolase-Familie 3 und entsprechend der Nomenklatur für Glykosid-Hydrolasen wurde ORF 1 als *xyl3A* und das entsprechende Protein als *Fg*Xyl3A benannt (Henrissat et al., 1998, FEBS Lett. 425(2):352-354).

Das für die $\beta$-Pyranosidase codierende Gen *xyl3A* besteht aus 2328 bp und codiert für das Protein *Fg*Xyl3A mit einer Größe von 775 Aminosäuren. *Fg*Xyl3A hat ein berechnetes Molekulargewicht von 85,9 Kilodalton (kDa) und einen theoretischen isoelektrischen Punkt (pl) von 5,55. Eine Signalsequenz konnte mittels "SignalP 3.0" nicht ermittelt werden.

Die Aminosäuresequenz von *Fg*Xyl3A ist in SEQ ID NO.1 gezeigt.

Ein Sequenzvergleich von *Fg*Xyl3A mit Sequenzen bekannter $\beta$-Xylosidasen wurde mit Hilfe von "blastp" der NCBI-Datenbank durchgeführt. Die Aminosäuresequenz von *Fg*Xyl3A zeigte die höchste Übereinstimmung zu einer putativen $\beta$-Xylosidase der Glykosid-Hydrolase-Familie 3 aus *Thermotoga neapolitana* mit einer Identität von 68%. In der Sequenz von *Fg*Xyl3A sind die konservierten Aminosäuren zu finden, die für Proteine der Glykosid-Hydrolase-Familie 3 charakteristisch sind (Zverlov et al., 1997, Microbiology 143 (Pt 11):3537-3542).

Die Domänenstruktur von *Fg*Xyl3A wurde mit Hilfe von "InterProScan" (http://www.ebi,ac.uk/Tools/InterProScan/) ermittelt. Das Protein *Fg*Xyl3A besteht demnach aus zwei Domänen, einer N-terminalen katalytischen Domäne und einer C-terminalen Domäne, wobei beide Domänen Ähnlichkeiten zur Glykosid-Hydrolase-Familie 3 zeigen (Abbildung 1).

Die SEQ ID NO. 1 enthält einen Bereich, der die beiden durch einen Sequenzvergleich mit dem Protein BglB aus *Thermotoga neapolitana* (Pozzo et al., 2010, J. Mol. Biol., 2:397(3), 724-739) ermittelten Strukturdomänen umfasst. Die Strukturdomäne 1 enthält die Aminosäurereste Leu13 bis Ile381 sowie Gln586 bis Tyr638 der SEQ ID NO.1 und wurde durch einen Sequenzvergleich mittels InterProScan mit SEQ ID NO. 4 ermittelt. Darin ist ein konservierter Aspartatrest vorhanden (Asp281), der als katalytisches Nucleophil bei Glykosid-Hydrolasen der Familie 3 identifiziert wurde (Zverlov et al., 1997, Microbiology 143 (Pt 11):3537-3542; Wulff-Strobel & Wilson, 1995, J. Bacteriol. 177(20):5884-5890).

Die Strukturdomäne 2 enthält die Aminosäurereste Val382 bis Val585 der SEQ ID NO.1 und wurde durch einen Sequenzvergleich mittels InterProScan mit SEQ ID NO. 4 ermittelt. SEQ ID NO. 2 ist derjenige Bereich von SEQ ID NO. 1, der beide Strukturdomänen ein und umfasst daher die Aminosäurereste 13 bis 638 der SEQ ID NO. 1.

Die zugrundeliegende Nucleotidesquenz des Vollängen-Protiens FgXyl3a wird durch SEQ ID NO.3 wieder gegeben.

**Beispiel 2: Klonierung der $\beta$-Pyranosidase *Fg*Xyl3A aus *Fervidobacterium grondwanense***

Für die Klonierung von *xyl3A* wurde das Gen mittels der folgenden Oligonucleotide mittels Polymerase-Kettenreaktion (polymerase chain reaction, PCR) nach Sambrook et al. (Molecular cloning, a laboratory manual, 2nd edition, 1989. Cold Spring Harbor Laboratory Press, Cold Spring Harbor, New York) amplifiziert:

| | |
|---|---|
| *xyl3A_Bam*HI.F | 5'-GGGGATCCGAGATATATAAGGATTCTTC-3' |
| *xyl3A_Hind*III.R | 5'-GGAAGCTTTAGAAAGTGTAAACTTTTG-3' |

Die Oligonucleotide enthielten am 5'-Ende eine *Bam*HI- und am 3'-Ende eine *Hind*III-Schnittstelle. Die PCR wurde in einem Thermocycler (Gene Amp PCR System 2400, Perkin-Elmer, Massachusetts, USA) durchgeführt und PCR-Produkte wurden mit dem "High Pure PCR Product Purification Kit" (Roche Diagnostics, Mannheim) nach Herstellerangaben gereinigt. Das amplifizierte Gen wurde mit Hilfe der durch die High Fidelity-Polymerase gebildeten 3'A-Überhänge in den Vektor pJET1.2 ("CloneJET™ PCR Cloning Kit" (Fermentas, St. Leon-Rot)) nach Angaben des Herstellers ligiert. Durch Restriktion mit den Endonucleasen *Bam*HI und *Hind*III wurde das Gen *xyl3A* aus dem Vektor geschnitten und in den ebenfalls mit den gleichen Restriktionsenzymen geschnittenen Vektor pQE-30 (Ampicillin-Resitenz, Expressionsvektor mit Sequenz für N-terminalen 6x Histidin-Tag mit T5-Promotor, Qiagen, Hilden) ligiert. Die korrekte Klonierung von *xyl3A* in den Vektor pQE-30 wurde durch Sequenzierung überprüft. Das rekombinante Plasmid pQE-30::xyl3A (Abbildung 2) wurde für die Genexpression in *E. coli* Stamm M15[pREP4] ($Nal^S$ $Str^S$ $Rif^S$ $Thi^-$ Lac- $Ara^+$ $Gal^+$ $Mtl^-$ $F^-$ $RecA^+$, $Uvr^+$ $Lon^+$ pREP4, Qiagen Hilden) transformiert. Durch die Klonierung von *xyl3A* in den pQE-30-Vektor wurde dem Gen am 5'-Ende eine für einen His-Tag codierende Sequenz angefügt. Somit ergibt sich für das rekombinante Protein *Fg*Xyl3A eine Größe von 786 Aminosäuren und ein berechnetes Molekulargewicht von 87,2 kDa.

**Beispiel 3: Expression von Fg*xyl3A* in *Escherichia coli* (*E. coli*)**

Der rekombinante Klon von *Escherichia coli* (*E. coli*) M15/pQE-30::*xyl3A* (Beispiel 2) wurde bei 37°C inkubiert. Die Zellanzucht der *E. coli*-Stämme erfolgte in LB-Medium (10 g/l Trypton, 5 g/l Hefeextrakt, 10 g/l NaCl, pH 7,0) nach Sambrook et al. (Molecular cloning, a laboratory manual, 2nd edition, 1989, Cold Spring Harbor Laboratory Press, Cold Spring Harbor, New York).

Die Kultivierung von Stämmen mit plasmid- bzw. genomcodierter Antibiotikaresistenz erfolgte unter Selektionsdruck durch Zugabe des entsprechenden Antibiotikums. Die verwendete Antibiotikakonzentration entsprach den Empfehlungen von Sambrook et al. (Molecular cloning, a laboratory manual, 2nd edition, 1989, Cold Spring Harbor Laboratory Press, Cold Spring Harbor, New York). Die Kultivierung der Zellen erfolgte aerob bei 37°C auf einem Schüttler (Certomat R, B. Braun Biotech International, Messungen) bei 160 Umdrehungen pro Minute. Das Wachstum der Zellen wurde anhand der optischen Dichte (OD) bei einer Wellenlänge von 600 nm in einem Spektralphotometer (UV-1602, Shimadzu Deutschland, Duisburg) bestimmt. Die Induktion der Genexpression rekombinanter *E. coli*-Stämme erfolgte durch Zugabe von 1 mM Isopropyl-$\beta$-D-thiogalactopyranosid (IPTG) bei einer $OD_{600}$nm von 0,6-0,8. Es folgte die Inkubation der Zellen für 4,5 h bei 37°C und 160 Umdrehungen pro Minutenute und eine anschließende Zentrifugation der Zellen in einer Sorvall RC 5C Plus-Zentrifuge (Thermo Fisher Scientific, Langenselbold) für 20 Minuten bei 4221 x *g* und 4°C, wobei etwa 2,0 g Zellen (Feuchtgewicht) aus 500 ml Kulturvolumen erhalten wurden. Das Pellet wurde direkt für die Präparation von zellfreiem Rohextrakt (Beispiel 4) verwendet oder bei -20 °C gelagert.

**Beispiel 4: Herstellung eines Rohextrakts aus dem rekombinanten *E. coli*-Klon M15/pQE-30::*xyl3A***

Für die Gewinnung von Rohextrakt wurde das Zellpellet (Beispiel 3) zweimal in Lysepuffer (50 mM $NaH_2PO_4$, 300 mM NaCl, 10 mM Imidazol, pH 8,0) gewaschen und jeweils für 20 Minuten bei 4500 x *g* und 4°C zentrifugiert. Anschließend wurde das Pellet in Lysepuffer aufgenommen, wobei pro 1 g Zellen 5 ml Lysepuffer eingesetzt wurden. Es folgte der Zellaufschluss mittels Ultraschall (Branson Sonifier®, Danbury, Connecticut, USA), wobei jeweils 3 Zyklen für je 5 Minuten auf Eis durchgeführt wurden (Output Control: 50%, Duty Cycle: 5). Die Zelltrümmer wurden anschließend durch Zentrifugation für 20 Minuten bei 20200 x *g* und 4°C abgetrennt und der Überstand in ein neues Gefäß überführt. Optional erfolgte eine Hitzefällung des Rohextrakts bei 60 °C für 15 Minuten und eine Abtrennung der gefällten Proteine durch Zentrifugation für 20 Minuten bei 20200 x *g* bei 4°C. Die Lagerung des Rohextrakts erfolgte bei 4°C.

Der Rohextrakt wurde mittels Standardtest auf $\beta$-Xylosidase-Aktivität untersucht (Beispiel 7) und für die Reinigung eingesetzt (Beispiel 5).

**Beispiel 5: Reinigung von *Fg*Xyl3A**

Die mehrstufige Reinigung von *Fg*Xyl3A durch (1) Hitzefällung, (2) Affinitätschromatographie und (3) Gelfiltration führte zur fast vollständigen Reinigung des Proteins.

(1) Hitzefällung: Nach dem ersten Reinigungsschritt mittels Hitzefällung (Beispiel 4) wurde eine Ausbeute von 41,7% wurde erzielt (Tabelle 1).

(2) Affinitätschromatographie: Die weitere Reinigung *Fg*Xyl3A mit angefügtem His-Tag (Beispiele 2, 3 und 4) erfolgte durch Affinitätschromatographie mittels Ni2+-Nitrilotriessigsäure ($Ni^{2+}$-NTA, Qiagen, Hilden). Zur Durchführung der Affinitätschromatographie wurde eine ÄKTA™ Purifier-Anlage (GE Healthcare, München) mit folgenden Komponenten verwendet: Monitor UV-900, Pumpe P-900, In-line Mixer M-925, Motor-Ventil INV-907 und Fraktionssammler Frac-950. Die verwendeten Puffer wurden vor der Nutzung filtriert (0,45 $\mu$m Cellulosemischester-Filter, Whatman, Dassel) und die Proteinproben für 10 Minuten bei 13000 x *g* und 4°C zentrifugiert. Nach der Equilibrierung der Säulenmatrix mit 5 Säulenvolumen Lysepuffer wurde der Rohextrakt bzw. hitzegefällter Rohextrakt mit einer Flussrate von 1 ml/Minuten auf die Säule geladen. Nichtgebundene Proteine wurden durch das Waschen der Säulenmatrix mit 3 Säulenvolumen Waschpuffer (50 mM $NaH_2PO_4$, 300 mM NaCl, 20 mM Imidazol, pH 8,0) und einer Flussrate von 2 ml/Minuten entfernt. Die Elution gebundener Proteine erfolgte durch das Spülen der Säule mit 5 Säulenvolumen Elutionspuffer (50 mM $NaH_2PO_4$, 300 mM NaCl, 250 mM Imidazol, pH 8,0) bei einer Flussrate von 2 ml/Minuten. Die Fraktionsgröße der gesammelten Proben betrug 2 ml. Die das Zielprotein enthaltenden Fraktionen wurden vereint, mit Hilfe von Mikrokonzentratoren (Amicon Ultra-15 Zentrifugalfitereinheit, 10 kDa Ausschlussgröße, Milipore, Schwalbach) aufkonzentriert und für weitere Reinigungsschritte eingesetzt bzw. dialysiert und für die biochemische Charakterisierung verwendet.
Diese weitere Reinigung mittels $Ni^{2+}$-NTA Agarose führte zu einem deutlichen Verlust der Ausbeute, die 1,13% betrug.

(3) Gelfiltration: Für die weitere Reinigung von *Fg*Xyl3A mit angefügtem His-Tag (Beispiele 2, 3 und 4) mittels Gelfiltration wurde eine ÄKTA™ Fast Protein Liquid Chromatography (FPLC)-Anlage (GE Healthcare, München) mit folgenden Komponenten verwendet: Monitor UPC-900, Pumpe P-920, In-line Mixer M-925, Motor-Ventil INV-907 und Fraktionssammler Frac-950. Die Reinigung mittels Gelfiltration erfolgte durch eine HiLoad 16/60 Superdex 200 prep grade Säule (GE Healthcare, München). Die Proteinproben wurden vor der Verwendung für 10 Minuten bei 13000 x *g* und 4°C zentrifugiert und die verwendeten Puffer filtriert (0,45 $\mu$m Cellulosemischester-Filter, Whatman, Dassel). Bei einer Flussrate von 1 ml/Minuten wurde die Säule mit maximal 1 ml Proteinprobe beladen und mit 1,5 Säulenvolumen 50 mM $Na_2HPO_4$ und 150 mM NaCl (pH 7,0) gespült. Dabei wurden Fraktionen in einer Größe von 2 ml gesammelt. Die Fraktionen, welche das Zielprotein enthielten, wurden vereint und mit Hilfe von Mikrokonzentratoren (Amicon Ultra-15 Zentrifugalfiltereinheit, 10 kDa Ausschlussgröße, Milipore, Schwalbach) aufkonzentriert. Die anschließende Entsalzung und Umpufferung von Proteinlösungen erfolgte durch Dialyse. Dazu wurde der Dialyseschlauch (Membra-Cel MWCO 3500, Serva, Heidelberg) in dem verwendeten Puffer gekocht und anschließend mit Proteinproben befüllt. Die Dialyse erfolgte in 50 mM Na-Acetat, pH 6,0 bzw. pH 6,5 über Nacht bei 4°C unter Rühren gegen das 100-fache Volumen der Proteinlösung. Nach der Dialyse wurden die Proben für die biochemische Charakterisierung eingesetzt. Durch den letzten Reinigungsschritt mittels Gelfiltration wurden eine Ausbeute von 0,18% und ein Reinigungsfaktor von 95 erzielt. Die spezifische Aktivität betrug dabei 20,9 U/mg gegenüber pNP-$\beta$-Xylopyranosid (Tabelle 1).

Die Aufreinigung von Histidin-getaggtem *Fg*Xyl3aist in Abbildung 3 und Tabelle 1 gezeigt.

| Reinigungsschritt | Gesamtprotein [mg] | Gesamtaktivität [U] | Spezifische Aktivität [U/mg] | Ausbeute [%] | Reinigungsfaktor [x-fach] |
|---|---|---|---|---|---|
| **Rohextrakt** | 1263,4 | 273,7 | 0,22 | 100 | 1 |
| **Hitzefällung** | 345,9 | 114,3 | 0,33 | 41,7 | 1.5 |
| **$Ni^{2+}$-NTA** | 8,1 | 3,1 | 0,38 | 1,13 | 1,7 |
| **Gelfiltration** | 0,015 | 0,32 | 20,9 | 0,18 | 95 |

Die Bestimmung von Proteinkonzentrationen erfolgte nach Bradford (1976, Anal Biochem 72:248-254). Dazu wurde 1 ml Bradford-Reagenz (0,007% (w/v) Serva Blau G-250, 5% (v/v) Ethanol, 8,5 % (v/v) H3PO4) mit 10 $\mu$l der Proteinprobe gemischt und 5 Minuten bei RT inkubiert. Die Extinktion wurde bei der Wellenlänge $\lambda$ = 595 nm spektrophotometrisch bestimmt. Als Standardprotein für die Erstellung von Kalibriergeraden wurde Rinderserumalbumin (BSA) in Konzentrationen von 0,1-1,0 mg/ml verwendet.

**Beispiel 6: Bestimmung des Molekulargewichts von *Fg*Xyl3A**

Die Bestimmung des Molekulargewichts von *Fg*Xyl3A erfolgte mittels denaturierender und nativer Polyacrylamidgelelektrophorese.

Die denaturierende Auftrennung von Proteinen erfolgte mittels Natriumdodecylsulfat-Polyacrylamidgelelektrophorese (SDS-PAGE) nach Laemmli (1970, Nature 227(5259):680-685). Als Proteinstandard wurde "Unstained Protein Molecular Weight Marker" bzw. "PageRuler™ Unstained Protein Ladder " von Fermentas (St. Leon-Rot) verwendet. Der Gel-Lauf wurde in einem "Minuteni-Protean Tetra"-System (Bio-Rad, München) in 25 mM Tris, 192 mM Glycin, 0,1% (w/v) SDS bei konstant 200 V durchgeführt. Die Gele wurden 30 Minuten in Coomassie- Färbelösung (0,25% (w/v) Serva Blau G-250, 45% (v/v) Ethanol, 10% (v/v) Essigsäure) inkubiert und anschließend in Entfärbelösung (30% (v/v) Ethanol, 10% (v/v) Essigsäure) entfärbt. Zur Konservierung wurden die Gele 10 Minuten in Trockenlösung (20% (v/v) Ethanol, 2% (v/v) Glycerin) inkubiert und mit Hilfe des "DryEase® Minuteni-Gel Drying System" (Invitrogen, Karlsruhe) getrocknet.

Die Ermittlung des Molekulargewichts von nativen Protein(komplex)en erfolgte durch native Polyacrylamidgelelektrophorese. Dazu wurden 4-12%ige (w/v) Tris-Glycin-Gradientengele (Anamed Elektrophorese, Groß-Bieberau) verwendet. Die Proteinproben wurden vor dem Lauf mit nativem Probenpuffer (7,5% (v/v) Glycerin, 0,04% (w/v) Bromphenolblau, 33 mM Tris, pH 6,8) versetzt. Als Proteinstandard diente "High Molecular Weight"-Marker (GE Healthcare, München). Der Gellauf wurde in einer "Novex® XCell II™ Minuteni-Cell"-Laufanlage (Invitrogen, Karlsruhe) für 3 h bei konstant 120 V in 25 mM Tris und 192 mM Glycin durchgeführt. Die Gele wurden 30 Minuten in Coomassie-Färbelösung inkubiert, entfärbt und zur Konservierung getrocknet.

Das rekombinante Protein zeigte unter denaturierenden Bedingungen ein Molekulargewicht von 87 kDa (Abbildung 4 A). Unter nativen Bedingungen wurde ein Molekulargewicht von 529,7 kDa ermittelt (Abbildung 4 B). Dies deutet darauf hin, dass das Protein FgXyl3A als Homohexamer vorliegen könnte.

**Beispiel 7: Aktivitätsnachweis von *Fg*Xyl3A in SDS-Gelen**

Der Nachweis enzymatischer Aktivität mit Hilfe von Zymogrammen erfolgte nach elektrophoretischer Auftrennung der Proteine durch SDS-PAGE (Beispiel 6). Die Gele wurden nach dem Lauf für 1 h in 1% (v/v) Triton X 100 bei Raumtemperatur (RT) inkubiert. Die $\beta$-Xylosidase-Aktivität von *Fg*Xyl3A wurde durch Inkubation der Gele in 0,1% (w/v) Esculin, 0,01% (w/v) Ammoniumeisen(III)-Citrat und 50 mM Na-Acetat (pH 6,0) durch Inkubation für 30-60 Minuten bei 60 °C nachgewiesen.

**Beispiel 8: Untersuchung der enzymatischen Aktivität von *Fg*Xyl3A**

Für die Bestimmung des Substratspektrums von *Fg*Xyl3A wurde die Aktivität des gereinigten rekombinanten Proteins (Beispiel 5) gegenüber pNP-$\beta$-D-Galaktopyranosid, pNP-$\alpha$-D-Glukopyranosid, pNP-$\beta$-D-Glukopyranosid, pNP-$\beta$-D-Cellobiosid, und pNP-$\beta$-D-Xylopyranosid untersucht. Die Substrate wurden in einer Endkonzentration von 2 mM eingesetzt und die Messung der Aktivitäten erfolgte mit Hilfe der folgenden Standardtests

(1) Bestimmung der $\beta$-Glukosidase-Aktivität: Die Aktivität der $\beta$-Glukosidase wurde in modifizierter Weise nach Park et al. (2005, Appl. Microbiol. Biotechnol. 69(4):411-422) mittels pNP-$\beta$-D-Glukopyranosid (Sigma-Aldrich, München) als Substrat bestimmt. Der Reaktionsansatz enthielt standardmäßig, sofern nicht anders aufgeführt, 2 mM pNP-$\beta$-DGlukopyranosid und 50 mM Na-Acetat (pH 6,0) in einem Gesamtvolumen von 1 ml. Vor Zugabe von 0,02 U Enzym wurden die Reaktionsansätze 5 Minuten bei 80 °C inkubiert. Die Reaktionen wurden für 10 Minuten bei 80 °C durchgeführt und durch Zugabe von 100 $\mu$l 0,1 M $Na_2CO_3$ und Überführen der Proben auf Eis gestoppt. Alle Messungen wurden in Dreifachbestimmung durchgeführt. Den Kontrollansätzen wurde das Enzym erst nach Zugabe von Na2CO3 und Inkubation auf Eis zugegeben. Die Bestimmung des freigesetzten p-Nitrophenols erfolgte bei einer Wellenlänge von $\lambda$ = 410 nm. Die enzymatische Aktivität wurde mit Hilfe der folgenden Formel berechnet:

$$\text{Enzymatische Aktivität (U/ml)} = (\Delta E \bullet V_{Ges}) / (d \bullet e \bullet t \bullet V_E)$$

Dabei ist $\Delta$E als Extinktionsänderung, $V_{Ges}$ als Reaktionsvolumen (ml), d als Küvettendicke in cm) e als molarer Extinktionskoeffizient (M-1 cm-1), t als Reaktionszeit (Minuten) und $V_E$ als Volumen der Enzymprobe (ml) definiert. Als molarer Extinktionskoeffizient wurde 16,56 M-1 cm-1 verwendet. 1 U $\beta$-Glukosidase-Aktivität wurde als die Enzymmenge definiert, die unter Standardbedingungen 1 $\mu$mol p-Nitrophenol pro Minutenute freisetzt.

(2) Bestimmung der $\beta$-Xylosidase-Aktivität: Für die Aktivitätsbestimmung der $\beta$-Xylosidase in modifizierter Weise nach Park et al. (2005, Appl. Microbiol. Biotechnol. 69(4):411-422) wurde pNP-$\beta$-D-Xylopyranosid (Sigma-Aldrich, München) als Substrat eingesetzt. Dazu erfolgte die Standardreaktion zur Bestimmung der $\beta$-Xylosidase-Aktivität, sofern nicht anders beschrieben, in Gegenwart von 2 mM pNP-$\beta$-D-Xylopyranosid und 50 mM Na-Acetat (pH 6,5) in einem Gesamtvolumen von 1 ml. Die Reaktionen wurden durch Zugabe von 0,016 U Enzym zu den für 5 Minuten bei 85 °C vorinkubierten Ansätzen gestartet und für 10 Minuten bei einer Temperatur von 85 °C durchgeführt. Durch Zugabe von 100 $\mu$l 0,1 M Na2CO3 und Überführen der Proben auf Eis wurden die Reaktionen gestoppt. Alle Messungen wurden in Dreifachbestimmung durchgeführt. Als Kontrolle dienten Reaktionsansätze, denen das Enzym nach der Zugabe von Na2CO3 und Inkubation auf Eis zugegeben wurde. Die spektrophotometrische Messung des freigesetzten p-Nitrophenols wurde bei einer Wellenlänge von $\lambda$ = 410 nm durchgeführt. Die Berechnung der $\beta$-Xylosidase-Aktivität erfolgte mittels folgender Formel:

$$\text{Enzymatische Aktivität (U/ml)} = (\Delta E \bullet V_{Ges}) / (d \bullet e \bullet t \bullet V_E)$$

Dabei ist $\Delta$E als Extinktionsänderung, $V_{Ges}$ als Reaktionsvolumen (ml), d als Küvettendicke (cm), e als molarer Extinktionskoeffizient (M-1 cm-1), t als Reaktionszeit (Minuten) und $V_E$ als Volumen der Enzymprobe (ml) definiert. Als molarer Extinktionskoeffizient wurde 16,56 M-1 cm-1 verwendet. 1 U $\beta$-Xylosidase-Aktivität wurde als die Enzymmenge definiert, welche unter Standardbedingungen zur Freisetzung von 1 $\mu$mol p-Nitrophenol pro Minutenute führte.

*Fg*Xyl3A zeigte die höchste spezifische Aktivität mit 20,9 U/mg gegenüber pNP-$\beta$-Xylopyranosid (Tabelle 2).

Weiterhin wurde eine spezifische Aktivität von 1,3 bzw. 9,3 U/mg gegenüber pNP-*α*- und pNP-*β*-Glukopyranosid bestimmt. Auch das Substrat pNP-*β*-Galaktopyranosid wurde durch die *β*-Xylosidase hydrolysiert, wobei die spezifische Aktivität 2,7 U/mg betrug. Eine Hydrolyse des Disaccharids pNP-*β*-Cellobiosid durch *Fg*Xyl3A wurde nicht nachgewiesen (Tabelle 2).

| Substrat | Spezifische Aktivität [U/mg] |
|---|---|
| pNP-β-Xylopyranosid | 20,9 |
| pNP-β-Glukopyranosid | 9,3 |
| pNP-α-Glukopyranosid | 1,3 |
| pNP-β-Galaktopyranosid | 2,7 |
| pNP-β-Cellobiosid | 0 |

**Tabelle 2: Substratspezifität von *FgXyl3A***

**Beispiel 9: Temperatureinfluss auf die Aktivität von *Fg*Xyl3A**

Zur Bestimmung des Temperaturprofils von *Fg*Xyl3A wurde die enzymatische Aktivität des rekombinanten Proteins (Beispiel 5) in einem Temperaturbereich von 10-115 °C mit pNP-*β*-Xylopyranosid als Substrat wie in Beispiel 8 beschrieben, gemessen. Die Reaktionsansätze wurden dafür vor Zugabe des Enzyms für 5 Minuten bei der entsprechenden Temperatur vorinkubiert. Die Ermittlung der Aktivität erfolgte mittels des Standardtests (Beispiel 8).

FgXyl3A zeigte in einem breiten Temperaturbereich *β*-Xylosidase-Aktivität (Abbildung 5). Mehr als 50% der relativen Aktivität wurden bei Temperaturen von 70-95 °C ermittelt, wobei die maximale Aktivität bei 85 °C gemessen wurde. Bei Temperaturen über 95 °C wurde ein starker Aktivitätsabfall beobachtet, so betrug die Aktivität von *Fg*Xyl3A bei 100 °C 23% der Maximalaktivität. In einem Temperaturbereich von 50-65 °C wurden 18-38% der relativen Aktivität gemessen.

**Beispiel 10: Temperaturstabilität von *Fg*Xyl3A**

Für die Bestimmung der Temperaturstabilität wurde rekombinante *Fg*Xyl3A (Beispiel 5) bei Temperaturen von 60-90 °C bis zu 24 h inkubiert. Die Bestimmung der Restaktivität erfolgte unter Standardbedingungen (Beispiel 8).

*Fg*Xyl3A zeigte bei einer Temperatur von 60 °C über einen Zeitraum von 3 h Stabilität, gemessen der *β*-Xylosidase-Restaktivität. Eine Inkubation bei 70-90 °C hingegen führte nach wenigen Minutenuten zu einer Inaktivierung des Enzyms (Abbildung 6). Durch die Vorinkubation von *Fg*Xyl3A bei 60 °C wurden nach 2 h 65% Restaktivität ermittelt, nach 6 h Inkubation betrug die Aktivität 30%. Nach einer längeren Inkubation für 24 h zeigte das Enzym eine Restaktivität von 25%. Bei einer Temperatur von 70 °C zeigte *Fg*Xyl3A nach der Inkubation für 5 Minuten 30% Restaktivität und nach 10 Minuten Vorinkubation betrug diese 15%. Die Halbwertzeit von *Fg*Xyl3A betrug bei 60 °C 215 Minuten und bei 70 °C 6 Minuten.

**Beispiel 11: Einfluss des pH-Wertes auf die Aktivität von *Fg*Xyl3A**

Zur Bestimmung des pH-Profils von FgXyl3A wurde die Aktivität des rekombinanten Enzyms (Beispiel 5) in einem pH-Bereich von 2,0-11,0 mit pNP-*β*-Xylopyranosid als Substrat ermittelt (Beispiel 8). Die Reaktionen erfolgten in Gegenwart von 50 mM
Universalpuffer (16,7 mM H3PO4, 16,7 mM Essigsäure, 16,7 mM H3BO4, Britton & Robinson, 1931) unter Standardbedingungen (Beispiel 8).

Die maximale Aktivität von *Fg*Xyl3A gegenüber *β*-Xylosidase wurde bei einem pH-Wert von 6,5 gemessen (Abbildung 7). In dem pH-Bereich von 5,5-7,0 zeigte die *β*-Xylosidase mehr als 45% der relativen Aktivität, wobei ein starker Aktivitätsabfall bei pH-Werten über 7,0 ermittelt wurde. Im sauren pH-Bereich zeigte das Enzym bei pH 2,0-3,0 keine Aktivität und bei pH 4,0 eine Maximalaktivität von 6%. Bei pH 5,0 wurde eine relative Aktivität von 36% gemessen.

**Beispiel 12: Bestimmung der pH-Stabilität von *Fg*Xyl3A**

Zur Bestimmung der pH-Stabilität wurde rekombinantes *Fg*Xyl3A (Beispiel 5) in 50 mM Universalpuffer mit pH-Werten von 3,0-10,0 für 48 h bei Raumtemperatur inkubiert. Es folgte die Bestimmung der Restaktivität unter Standardbedingungen (Beispiel 8), jedoch in Gegenwart von 50 mM Universalpuffer mit einem pH-Wert von 6,5.

Im leicht sauren und neutralen pH-Bereich von 5,0-7,0 zeigte *Fg*Xyl3A eine hohe Stabilität mit einer Restaktivität gegenüber β-Xylosidase von 90-95% (Abbildung 8). Im sauren pH-Bereich hingegen kam es zu einem Aktivitätsverlust, so betrug die Restaktivität 66% bei pH 3,0 und 4,0. Ein geringer Verlust der Aktivität wurde im alkalischen pH-Bereich beobachtet. Hier wurde eine Restaktivität von 87% bei pH 8,0, 81% bei pH 9,0 und 80% bei pH 10,0 ermittelt.

**Beispiel 13: Produktanalyse durch Hochleistungsflüssigkeitschromatographie (HPLC)**

Zum Nachweis der gebildeten Hydrolyseprodukte wurden 0,5% (w/v) Buchenholz-Xylan, Xylobiose, Xylotriose und Xylotetraose in 50 mM $NaH_2PO_4$ (pH 6,5) mit 0,1 U β-Xylosidase bis zu 3 h bei 80 °C inkubiert. Die Proben wurden zum Beenden der Reaktion für 10 Minuten gekocht und anschließend für 10 Minuten bei 13000 x g und 4°C zentrifugiert. Der Überstand der Proben wurde filtriert (0,2 μm Porengröße, Pall, Darmstadt) und in Probenflaschen mit Mikroeinsätzen (CS Chromatographie Service, Langerwehe) überführt. Von jeder Probe wurden 20 μl injiziert. Die Analyse der Produkte erfolgte mit Hilfe einer Aminex HPX 42-A-Säule (Bio-Rad, München), wobei als mobile Phase entgastes $H_2O$ (LiCrosolv®, Merck, Darmstadt) eingesetzt und eine Flussrate von 0,6 ml/Minuten bei einer Temperatur von 70 °C gewählt wurde.

Nach Inkubation des Enzyms für 3 h in Gegenwart von Xylobiose wurde ein Großteil des Substrats zu Xylose hydrolysiert (Abbildung 9). Die Inkubation von *Fg*Xyl3A mit Xylotriose führte zur Bildung von Xylose und Xylobiose, wobei ein großer Teil des Substrats hydrolysiert wurde (Abbildung 10). Wurde *Fg*Xyl3A mit dem Substrat Xylotetraose inkubiert, so wurden die Hydrolyseprodukte Xylose, Xylobiose und Xylotriose detektiert, wobei Xylose das Hauptprodukt war (Abbildung 11). Eine längere Inkubation führte nicht zu einem erhöhten Abbau von Xylotetraose. Erfolgte die Inkubation von *Fg*Xyl3A in Gegenwart von Buchenholz-Xylan, so wurden geringe Mengen an Xylose als Hydrolyseprodukt detektiert.

**Beispiel 14: Kinetik von *Fg*Xyl3A**

Die Bestimmung der kinetischen Parameter der β-Xylosidase-Aktivität von *Fg*Xyl3A erfolgte mit Substratkonzentrationen von 0,01-6,0 mM pNP-β-D-Xylopyranosid. Die Reaktionsdurchführung erfolgte unter Standardbedingungen (Beispiel 8). Die Ermittlung von $v_{max}$ und $K_M$ erfolgte mittels nichtlinearer Regression nach Michaelis-Menten und ergab einen $v_{max}$-Wert von 0,53 μmol/min und einen $K_M$-Wert von 0,06 mM (Abbildung 12).

**Übersicht über die Sequenzen in der Sequenzliste:**

SEQ ID NO.1: FgXyl3A Volllängen-Protein

SEQ ID NO.2: Katalytische Domäne GHF 3 aus SEQ ID NO.1 (entspricht Aminosäurerest 13 bis 638 aus SEQ ID NO.1)

SEQ ID NO. 3: DNA-Sequenz, die für SEQ ID NO. 1 codiert

SEQ ID NO. 4: Gersten-β-D-Glukan-Exohydrolase-Isoenzym Exo I, Volllänge

**Kurze Beschreibung der Zeichnungen**

**Abbildung 1: Domänenstruktur von *Fg*Xyl3A**

Dargestellt ist die mittels "InterProScan" ermittelte Domänenstruktur der β-Xylosidase *Fg*Xyl3A. GHF: Glykosid-Hydrolase-Familie.

**Abbildung 2: Vektorkarte des rekombinanten Plasmids pQE-30::*xyl3A***

Das für die β-Xylosidase *Fg*Xyl3A codierende Gen *xyl3A* wurde in den Expressionsvektor pQE-30 mit Hilfe der Restriktionsschnittstellen *Bam*HI und *Hind*III ligiert. T5: T5-Promotor, *bla:* β-Lactamase, ColE1: Replikationsursprung.

**Abbildung 3: Reinigung und Aktivitätsnachweis von *Fg*Xyl3A**

Der Nachweis der Reinigung und Aktivität von *Fg*Xyl3A erfolgte mittels SDS-PAGE. Die Proteine wurden mit Coomassie-Blau gefärbt, die Aktivität von *Fg*Xyl3A wurde durch Esculin detektiert. Spur 1: Proteinstandard "PageRuler™ Unstained Protein Ladder" (Fermentas, St. Leon-Rot), Spur 2: Rohextrakt von *E. coli* M15/pQE-30 (Kontrolle, 35 $\mu$g), Spur 3: Rohextrakt von *E. coli* M15/pQE-30*::xyl3A* (37 $\mu$g), Spur 4: hitzegefällter Rohextrakt von *E. coli* M15/pQE-30::*xyl3A* (35 $\mu$g), Spur 5: $Ni^{2+}$-NTA Fraktion von *Fg*Xyl3A (40 $\mu$g), Spur 6: Gelfiltrationsfraktion von FgXyl3A (1,1 $\mu$g), Spur 7: Aktivitätsgel der Gelfiltrationsfraktion von *Fg*Xyl3A (1,1 $\mu$g).

**Abbildung 4: Bestimmung des Molekulargewichts von *Fg*Xyl3A**

Die Bestimmung des Molekulargewichts von *Fg*Xyl3A erfolgte mittels denaturierender und nativer Polyacrylamidgelelektrophorese. Dargestellt ist die Auftragung des Logarithmus der Molekulargewichte gegen die Laufstrecke der Proteine im Gel (Rf-Wert). Molekulargewichte der Standardproteine in (A): Rekombinante Proteine mit einer Größe von 200, 150, 120, 100, 85, 70 und 60 kDa. Molekulargewichte der Standardproteine in (B): Thyroglobulin (669 kDa), Ferritin (440 kDa), Katalase (232 kDa), Lactatdehydrogenase (140 kDa), Albumin (66 kDa).

**Abbildung 5: Temperaturprofil von *Fg*Xyl3A**

Die Bestimmung der Aktivität von *Fg*Xyl3A erfolgte in einem Temperaturbereich von 10-115°C unter Standardbedingungen. Reaktionsparameter: 2 mM pNP-$\beta$-Xylopyranosid, 50 mM Na-Acetat, pH 6,5; 10 min Inkubationszeit.

**Abbildung 6: Temperaturstabilität von *Fg*Xyl3A**

Die Bestimmung der Temperaturstabilität von FgXyl3A erfolgte nach Vorinkubation des Enzyms bei Temperaturen von 60-100 °C. Reaktionsparameter: 2 mM pNP-$\beta$-Xylopyranosid, 50 mM Na-Acetat, pH 6,5, 85°C; 10 min Inkubationszeit.

**Abbildung 7: pH-Profil von *Fg*Xyl3A**

Die Bestimmung der Aktivität von *Fg*Xyl3A erfolgte in einem pH-Bereich von 2,0-11,0 unter Standardbedingungen. Reaktionsparameter: 2 mM pNP-$\beta$-Xylopyranosid, 50 mM Universalpuffer, 85°C; 10 min Inkubationszeit.

**Abbildung 8: pH-Stabilität von *Fg*Xyl3A**

Die Bestimmung der pH-Stabilität von *Fg*Xyl3A erfolgte nach Vorinkubation des Enzyms bei pH-Werten von 3,0-10,0 unter Standardbedingungen. Reaktionsparameter: 2 mM pNP-$\beta$-Xylopyranosid, 50 mM Universalpuffer, 85 °C; 10 Minuten Inkubationszeit.

**Abbildung 9: Hydrolyse von Xylobiose durch *Fg*Xyl3A**

Die Analyse der Hydrolyseprodukte erfolgte nach Inkubation von *Fg*Xyl3A für 3 h in Gegenwart von 0,5% (w/v) Xylobiose, 20 mM Na-Phosphatpuffer, pH 6,5, 80 °C mittels HPLC.

**Abbildung 10: Hydrolyse von Xylotriose durch *Fg*Xyl3A**

Die Analyse der Hydrolyseprodukte erfolgte nach Inkubation von *Fg*Xyl3A für 3 h in Gegenwart von 0,5% (w/v) Xylotriose, 20 mM Na-Phosphatpuffer, pH 6,5, 80 °C mittels HPLC.

**Abbildung 11: Hydrolyse von Xylotetraose durch *Fg*Xyl3A**

Die Analyse der Hydrolyseprodukte erfolgte nach Inkubation von *Fg*Xyl3A für 3 h in Gegenwart von 0,5% (w/v) Xylotetraose, 20 mM Na-Phosphatpuffer, pH 6,5, 80 °C mittels HPLC.

**Abbildung 12: Kinetik von *Fg*Xyl3A**

Dargestellt ist der Michaelis-Menten-Plot von *Fg*Xyl3A. Die Aktivität von *Fg*Xyl3A wurde unter Standardbedingungen ermittelt. Reaktionsparameter: 0,01-6,0 mM pNP-$\beta$-Xylopyranosid, 50 mM Na-Acetat, pH 6,5, 85 °C; 10 Minuten Inkubationszeit.

## Patentansprüche

1. Polypeptid mit [beta]-Pyranosidase-Aktivität, wobei das Polypeptid eine Aminosäuresequenz umfasst, die mindestens 75% Sequenzidentität mit SEQ ID NO. 2 hat.

2. Polypeptid mit [beta]-Pyranosidase-Aktivität, wobei das Polypeptid eine Aminosäuresequenz umfasst, die mindestens 75% Sequenzidentität mit SEQ ID NO. 1 hat.

3. Polypeptid nach einem oder mehreren der vorhergehenden Ansprüche, wobei die [beta]-Pyranosidase-Aktivität ausgewählt wird aus [beta]-Xylopyranosidase-Aktivität, [beta]-Glukopyranosidase-Aktivität und einer Kombination dieser beiden Aktivitäten.

4. Polypeptid nach einem oder mehreren der vorhergehenden Ansprüche, dessen Aktivität gegenüber pNP-[beta]-Cellobiosid 5% oder weniger ist, verglichen mit der Aktivität gegenüber pNP-[beta]-Xylobiosid.

5. Polypeptid nach einem oder mehreren der Ansprüche 2 bis 4, dessen enzymatischen Aktivität gegenüber pNP-[beta]-Xylopyranosid in einem Bereich von pH 4,0 bis pH 8,0, davon möglichst in einem Bereich von pH 5,5 bis 7,0, und vorzugsweise in einem Bereich von pH 6,2 bis 6,8 maximal ist.

6. Polypeptid nach einem oder mehreren der Ansprüche 2 bis 5, dessen maximale enzymatische Aktivität gegenüber pNP-[beta]-Xylopyranosid nach 48 Stunden Inkubation bei pH 9,0 mindestens 50% der Aktivität einer zweiten Fraktion dieses Polypeptids beträgt, die nicht bei pH 9,0 inkubiert wurde.

7. Fusionsprotein aus dem Polypeptid nach einem beliebigen der vorhergehenden Ansprüche und einer Kohlenhydrat-Bindedomäne eines anderen Proteins.

8. Fusionsprotein aus einem Polypeptid nach einem beliebigen der vorhergehenden Ansprüche und einem zweiten Polypeptid ausgewählt aus einem oder mehreren der aus der Gruppe bestehend aus Signalpeptid, Affinitäts-Tag, Protease-Schnittstelle.

9. Mischung, die das Polypeptid nach einem der vorhergehenden Ansprüche und eine oder mehrere Pektinasen und/oder eine oder mehrere Endoxylanasen und/oder eine oder mehrere [beta]-Glukosidasen und/oder eine oder mehrere [beta]-Glukanasen und/oder eine oder mehrere Cellobiohydrolasen und/oder eine oder mehrere [beta]-Xylosidasen und/oder eine oder mehrere [alpha]-Arabinofuranosidasen und/oder eine oder mehrere [alpha]-Glukuronidasen und/oder eine oder mehrere Acetylxylan-Esterasen enthält.

10. Nucleinsäure, die für ein Polypeptid nach einem der Ansprüche 1 bis 8 codiert.

11. Vektor, der die Nucleinsäure nach Anspruch 10 enthält.

12. Wirtszelle, die einen Vektor nach Anspruch 11 enthält.

**13.** Verfahren zur Aufreinigung eines Polypeptids mit [beta]-Pyranosidase-Aktivität einem der Ansprüche 1 bis 8, bestehend aus:

a) Gewinnen der Wirtszelle, die mit einem Vektor nach Anspruch 11 transformiert wurde
b) Aufzucht der Wirtszelle unter Bedingungen unter denen das Polypeptid mit [beta]-Pyranosidase-Aktivität exprimiert wird
c) Aufreinigung des Polypeptids mit [beta]-Pyranosidase-Aktivität.

**14.** Verfahren nach Anspruch 13, wobei c) eine Hitzefällung umfasst.

**15.** Verwendung des Polypeptids nach einem oder mehreren der Ansprüche 1 bis 8 oder der Mischung nach Anspruch 9 zum Abbau von einem oder mehreren der folgenden Substrate: [beta]-Xylopyranosid oder [beta]-Glukopyranosid.

## Claims

**1.** Polypeptide with [beta]-pyranosidase activity, wherein the polypeptide comprises an amino acid sequence which has at least 75% sequence identity with SEQ ID NO. 2.

**2.** Polypeptide with [beta]-pyranosidase activity, wherein the polypeptide comprises an amino acid sequence which has at least 75% sequence identity with SEQ ID NO. 1.

**3.** Polypeptide according to one or more of the preceding claims, wherein the [beta]-pyranosidase activity is selected from [beta]-xylopyranosidase activity, [beta]-glucopyranosidase activity and a combination of these two activities.

**4.** Polypeptide according to one or more of the preceding claims, the activity of which against pNP-[beta]-cellobioside is 5% or less, compared with the activity against pNP-[beta]-xylobioside.

**5.** Polypeptide according to one or more of Claims 2 to 4, the enzymatic activity of which against pNP-[beta]-xylopyranoside is a maximum in a range from pH 4.0 to pH 8.0, of which as far as possible in a range from pH 5.5 to 7.0, and preferably in a range from pH 6.2 to 6.8.

**6.** Polypeptide according to one or more of Claims 2 to 5, the maximum enzymatic activity of which against pNP-[beta]-xylopyranoside after 48 hours of incubation at pH 9.0 is at least 50% of the activity of a second fraction of said polypeptide that has not been incubated at pH 9.0.

**7.** Fusion protein of the polypeptide according to any one of the preceding claims and a carbohydrate-binding domain of a different protein.

**8.** Fusion protein of a polypeptide according to any one of the preceding claims and a second polypeptide selected from one or more of the group consisting of signal peptide, affinity tag, protease cleavage site.

**9.** Mixture which contains the polypeptide according to one of the preceding claims and one or more pectinases and/or one or more endoxylanases and/or one or more [beta]-glucosidases and/or one or more [beta]-glucanases and/or one or more cellobiohydrolases and/or one or more [beta]-xylosidases and/or one or more [alpha]-arabinofusanosidases and/or one or more [alpha]-glucuronidases and/or one or more acetylxylan esterases.

**10.** Nucleic acid which encodes a polypeptide according to one of Claims 1 to 8.

**11.** Vector which contains the nucleic acid according to Claim 10.

**12.** Host cell which contains a vector according to Claim 11.

**13.** Method for purifying a polypeptide with [beta]-pyranosidase activity according to one of Claims 1 to 8, consisting of:

a) obtaining the host cell that was transformed with a vector according to Claim 11
b) culturing the host cell under conditions under which the polypeptide with [beta]-pyranosidase activity is expressed

c) purifying the polypeptide with [beta]-pyranosidase activity.

**14.** Method according to Claim 13, wherein c) comprises a heat precipitation.

**15.** Use of the polypeptide according to one or more of Claims 1 to 8, or of the mixture according to Claim 9 for the degradation of one or more of the following substrates: [beta]-xylopyranoside or [beta]-glucopyranoside.

**Revendications**

**1.** Polypeptide ayant une activité de [bêta]-pyrannosidase, le polypeptide comprenant une séquence d'acides aminés qui a une identité de séquence d'au moins 75 % avec SEQ ID NO:2.

**2.** Polypeptide ayant une activité de [bêta]-pyrannosidase, le polypeptide comprenant une séquence d'acides aminés qui a une identité de séquence d'au moins 75 % avec SEQ ID NO:1.

**3.** Polypeptide selon l'une ou plusieurs des revendications précédentes, l'activité de [bêta]-pyrannosidase étant choisie parmi une activité de [bêta]-xylopyrannosidase, une activité de [bêta]-glucopyrannosidase, ou une combinaison de ces deux activités.

**4.** Polypeptide selon l'une ou plusieurs des revendications précédentes, dont l'activité vis-à-vis du pNP-[bêta]-cellobioside est de 5 % ou moins, par comparaison avec l'activité vis-à-vis du pNP-[bêta]-xylobioside.

**5.** Polypeptide selon l'une ou plusieurs des revendications 2 à 4, dont l'activité enzymatique vis-à-vis du pNP-[bêta] -xylopyrannoside est maximale dans une plage de pH 4,0 à pH 8,0, dans cette dernière si possible dans une plage de pH 5,5 à 7,0, et de préférence dans une plage de pH 6,2 à 6,8.

**6.** Polypeptide selon l'une ou plusieurs des revendications 2 à 5, dont l'activité enzymatique maximale vis-à-vis du pNP-[bêta]-xylopyrannoside est, après 48 heures d'incubation à pH 9,0, d'au moins 50 % de l'activité d'une deuxième fraction de ce polypeptide, qui n'a pas été incubée à pH 9,0.

**7.** Protéine de fusion constituée du polypeptide selon l'une quelconque des revendications précédentes et d'un domaine de liaison aux hydrates de carbone d'une autre protéine.

**8.** Protéine de fusion constituée d'un polypeptide selon l'une quelconque des revendications précédentes et d'un deuxième polypeptide choisi parmi un ou plusieurs de ceux du groupe consistant en un peptide signal, une étiquette d'affinité, un site de clivage par une protéase.

**9.** Mélange qui contient le polypeptide selon l'une ou plusieurs des revendications précédentes et une ou plusieurs pectinases et/ou une ou plusieurs endoxylanases et/ou une ou plusieurs [bêta]-glucosidases et/ou une ou plusieurs [bêta]-glucanases et/ou une ou plusieurs cellobiohydrolases et/ou une ou plusieurs [bêta]-xylosidases et/ou une ou plusieurs [alpha]-arabinofurannosidases et/ou une ou plusieurs [alpha]-glucuronidases et/ou une ou plusieurs acétylxylane-estérases.

**10.** Acide nucléique qui code pour un polypeptide selon l'une des revendications 1 à 8.

**11.** Vecteur qui contient l'acide nucléique selon la revendication 10.

**12.** Cellule hôte qui contient un vecteur selon la revendication 11.

**13.** Procédé de purification d'un polypeptide ayant une activité de [bêta]-pyrannosidase selon l'une des revendications 1 à 8, consistant en :

a) l'obtention de la cellule hôte, qui a été transformée par un vecteur selon la revendication 11,
b) la culture de la cellule hôte dans des conditions dans lesquelles le polypeptide ayant une activité de [bêta] -pyrannosidase est exprimé,
c) purification du polypeptide ayant une activité de [bêta]-pyrannosidase.

**14.** Procédé selon la revendication 13, dans lequel c) comprend une précipitation par chauffage.

**15.** Utilisation du polypeptide selon l'une ou plusieurs des revendications 1 à 8 ou du mélange selon la revendication 9 pour dégrader un ou plusieurs des substrats suivants : [bêta]-xylopyrannoside ou [bêta]-glucopyrannoside.

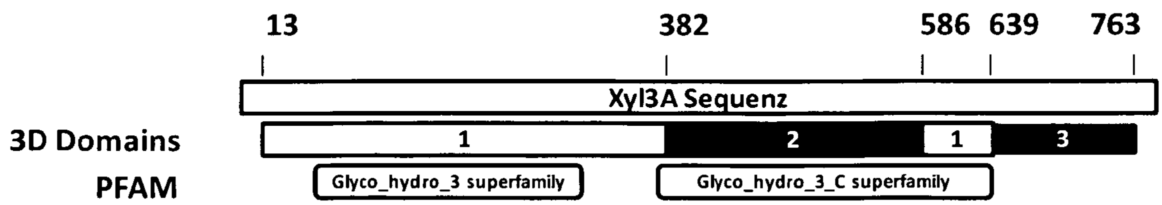
13
382
586
639
763
Xyl3A Sequenz
3D Domains
1
2
1
3
PFAM
Glyco_hydro_3 superfamily
Glyco_hydro_3_C superfamily

Abbildung 1

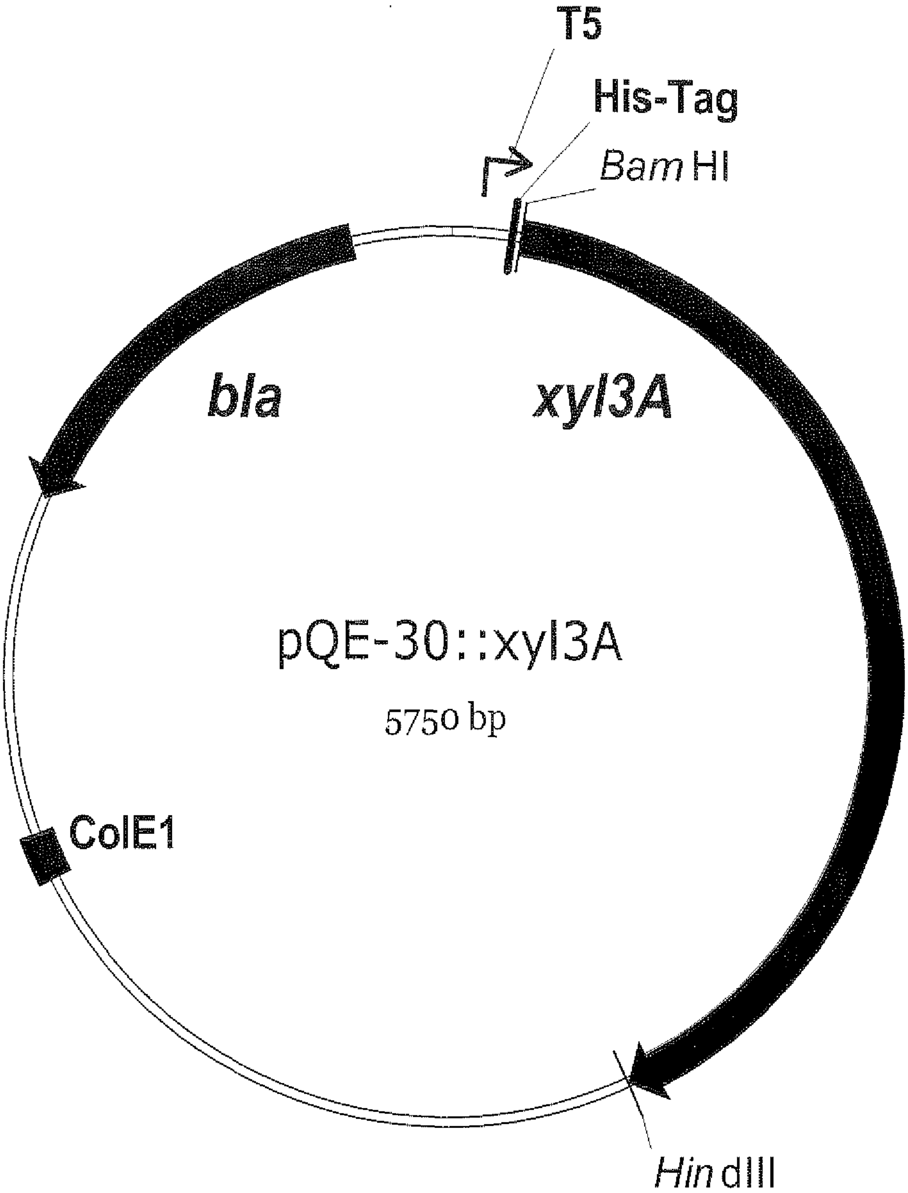


Abbildung 2

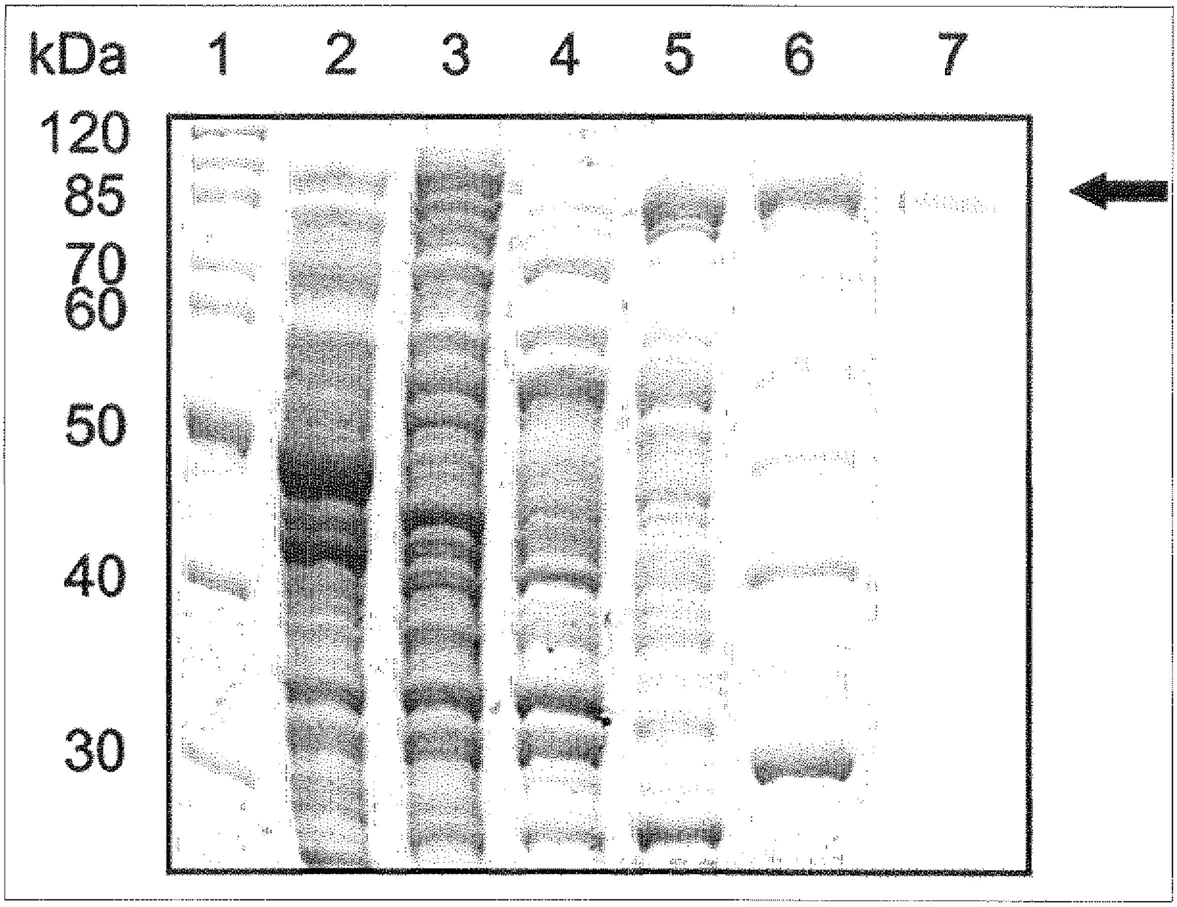


Abbildung 3

A

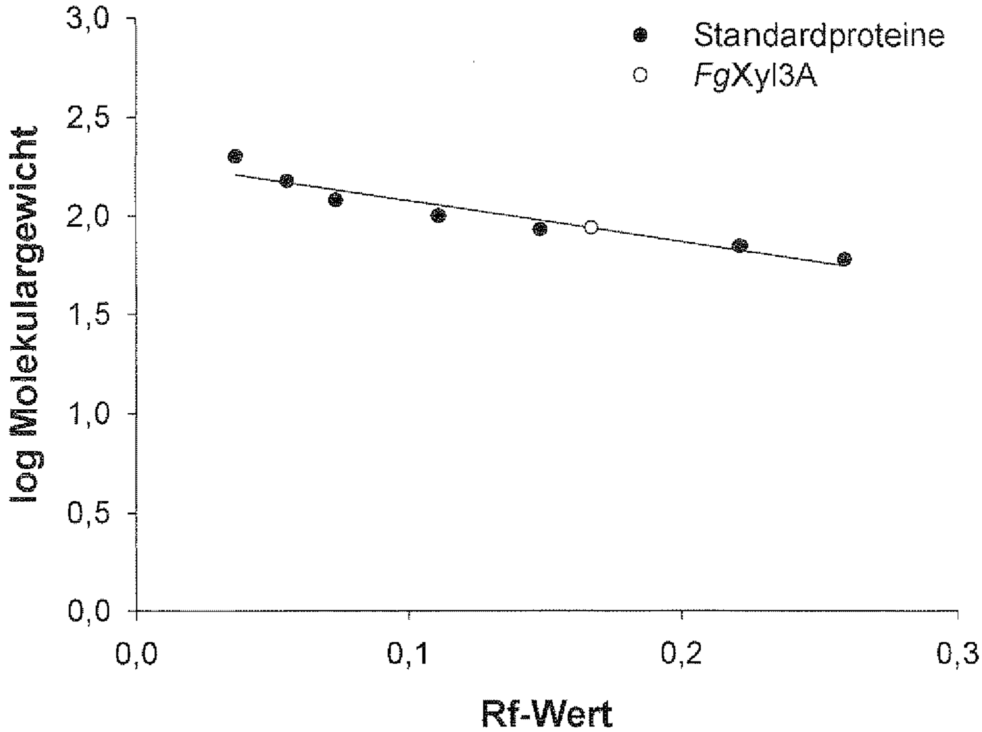


B

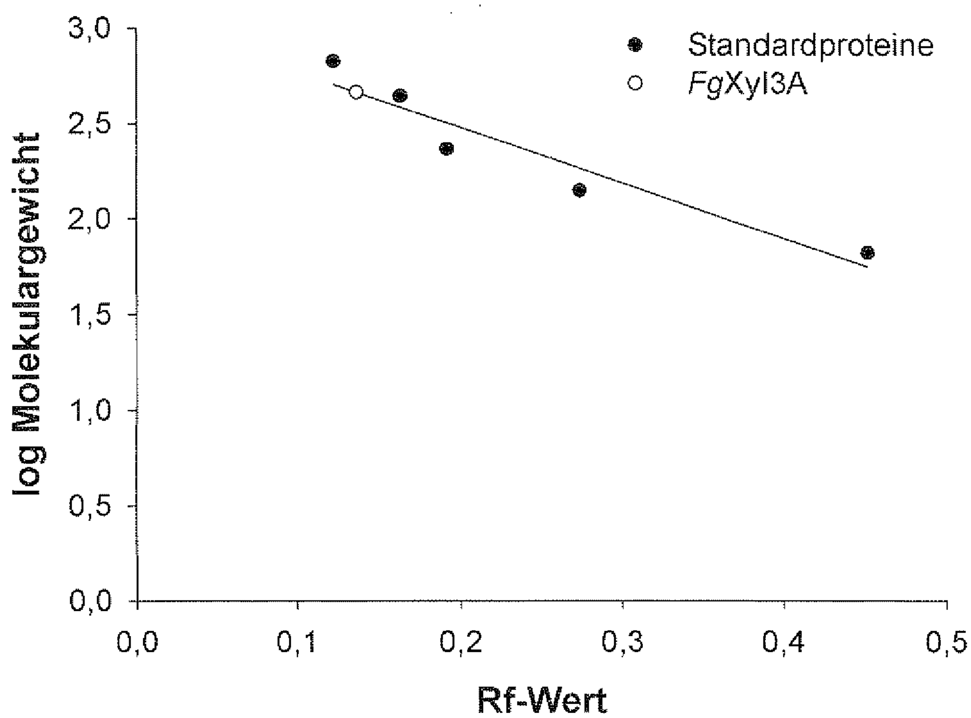


Abbildung 4

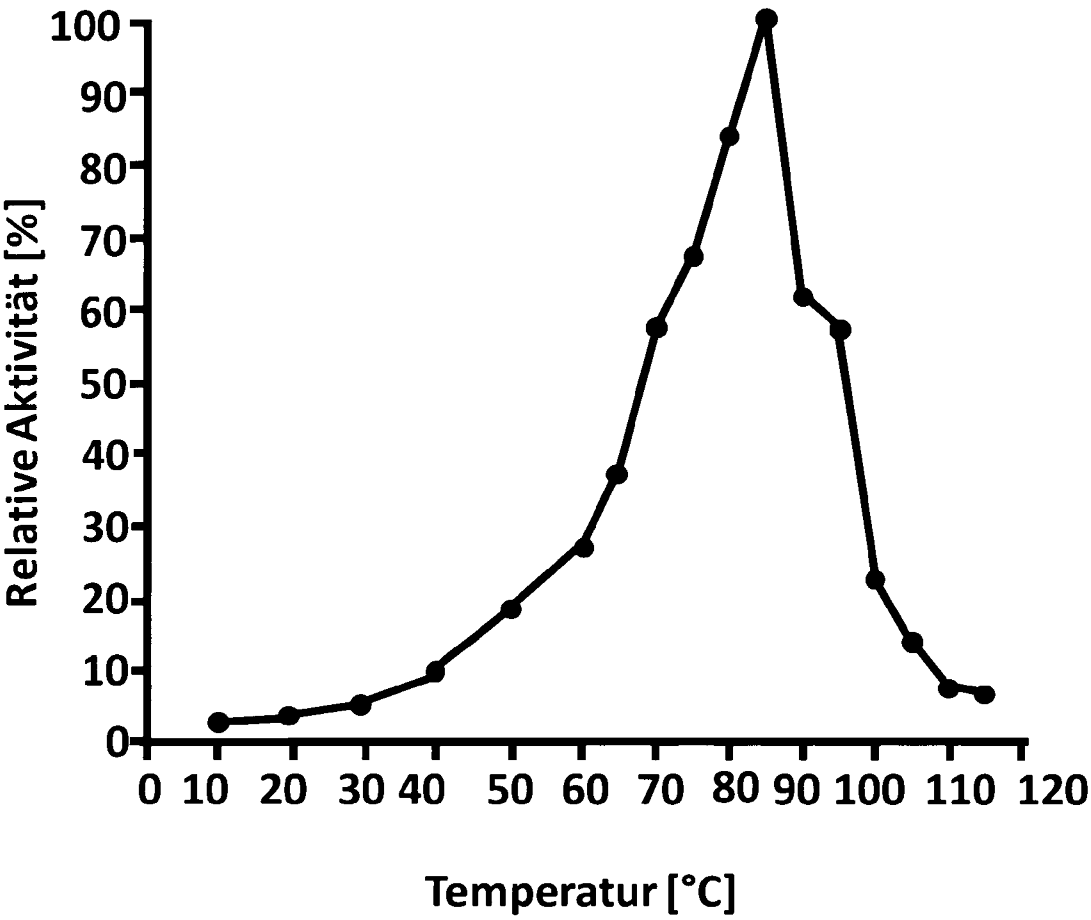
100
90
80
70
60
50
40
30
20
10
0
Relative Aktivität [%]
0 10 20 30 40 50 60 70 80 90 100 110 120
Temperatur [°C]

Abbildung 5

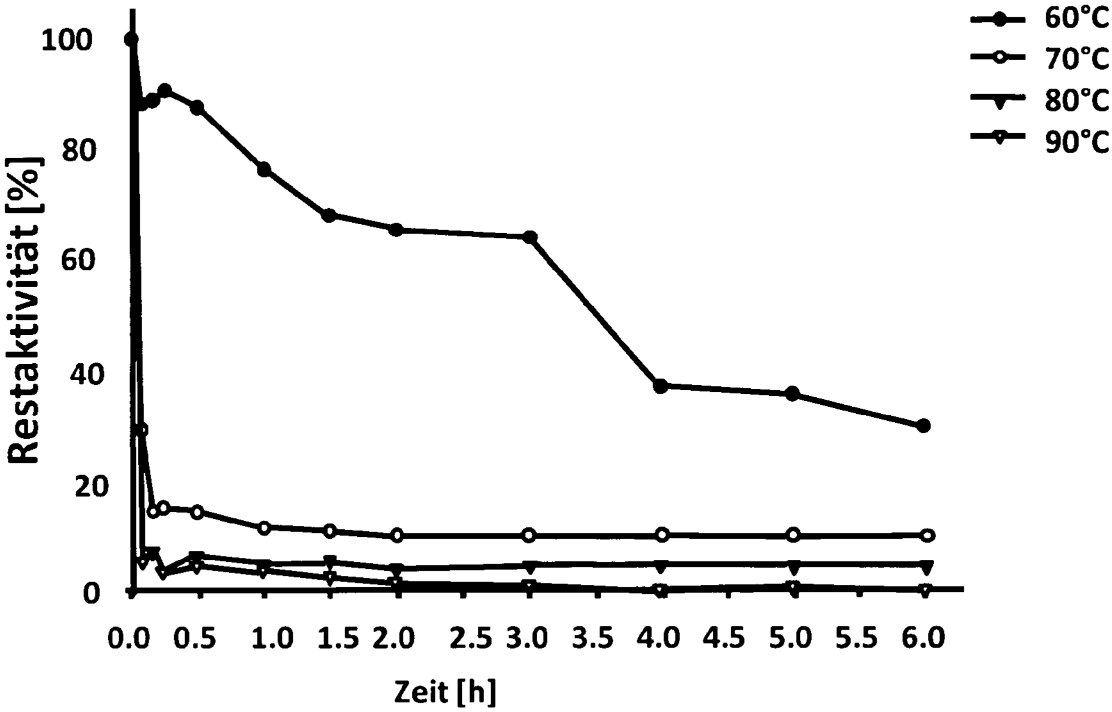
60°C
70°C
80°C
90°C
100
80
60
40
20
0
Restaktivität [%]
0.0 0.5 1.0 1.5 2.0 2.5 3.0 3.5 4.0 4.5 5.0 5.5 6.0
Zeit [h]

Abbildung 6

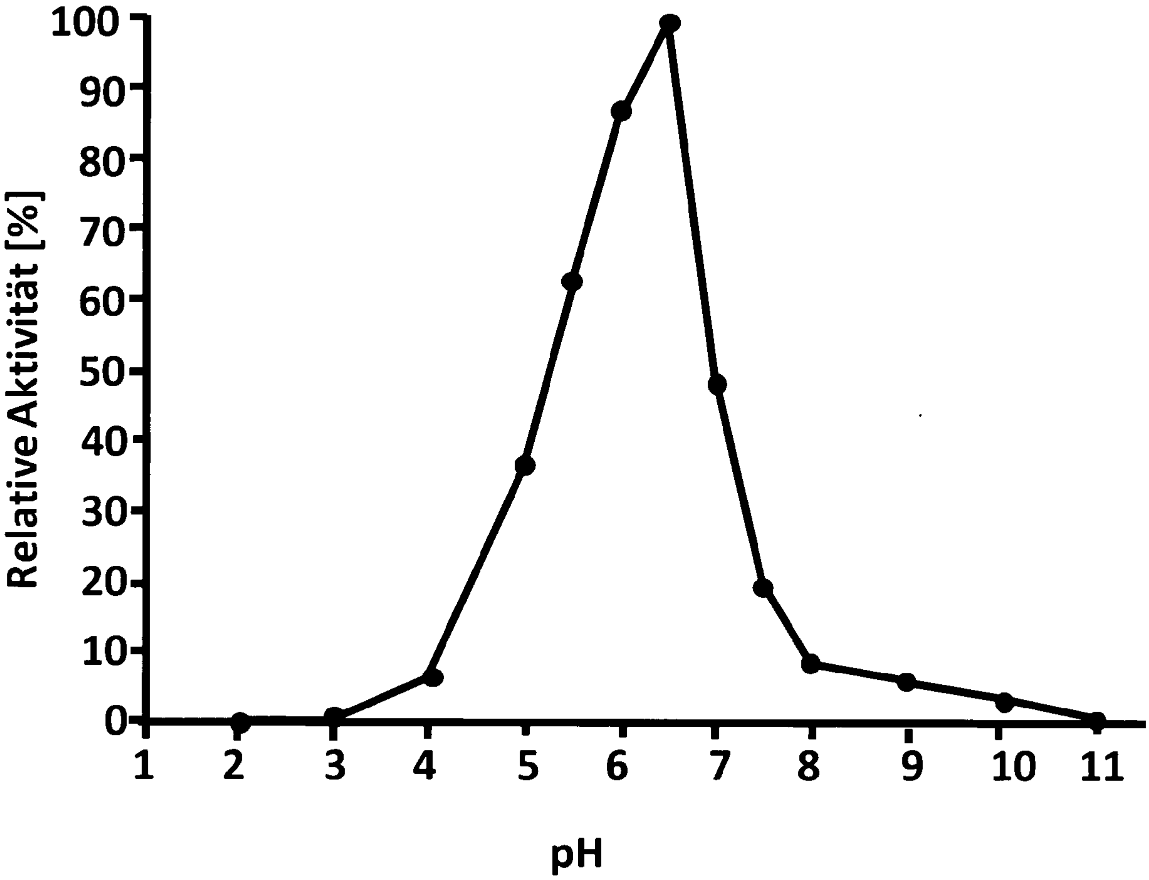
Relative Aktivität [%]
100
90
80
70
60
50
40
30
20
10
0
1
2
3
4
5
6
7
8
9
10
11
pH

Abbildung 7

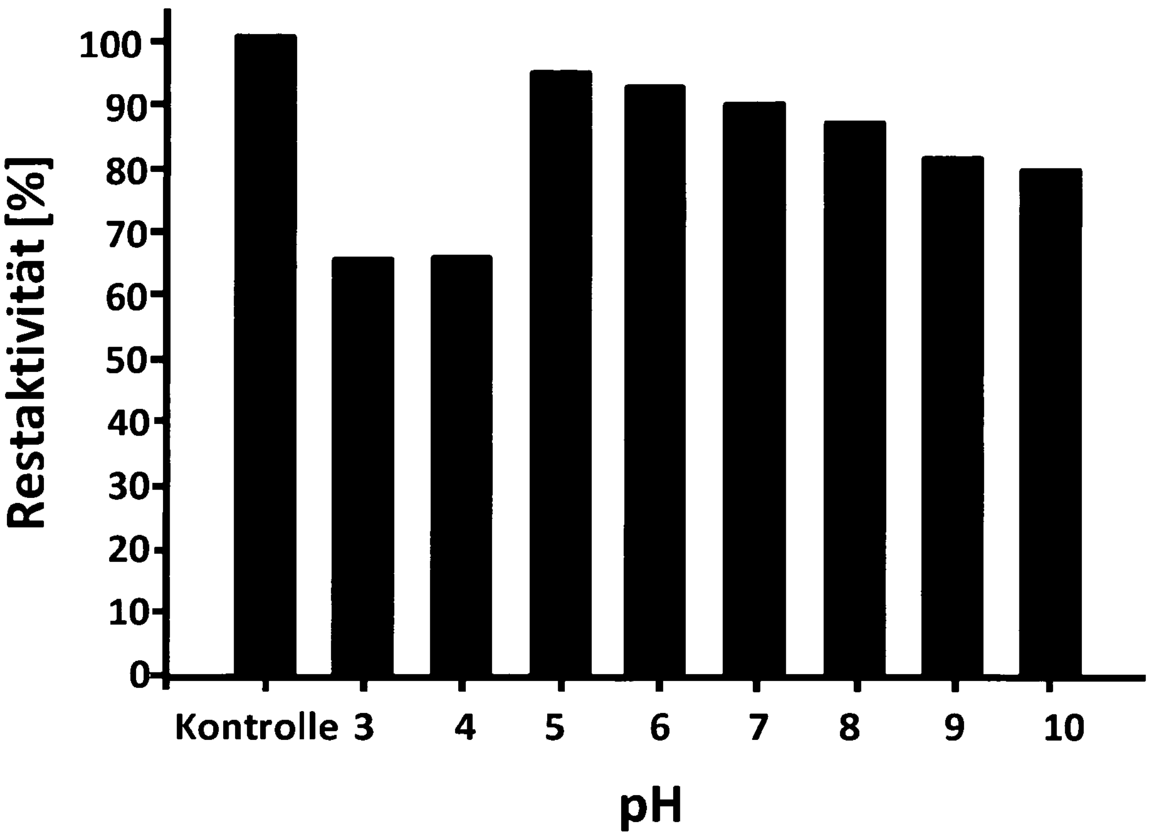
Restaktivität [%]
100
90
80
70
60
50
40
30
20
10
0
Kontrolle 3
4
5
6
7
8
9
10
pH

Abbildung 8

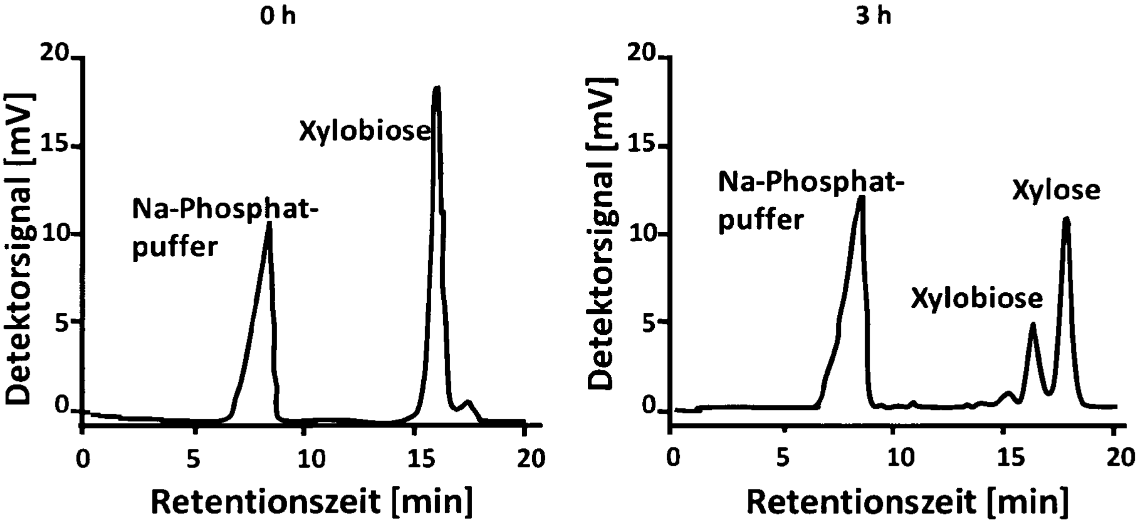
0 h
Detektorsignal [mV]
20
15
10
5
0
Xylobiose
Na-Phosphat-
puffer
0
5
10
15
20
Retentionszeit [min]
3 h
Detektorsignal [mV]
20
15
10
5
0
Na-Phosphat-
puffer
Xylose
Xylobiose
0
5
10
15
20
Retentionszeit [min]

Abbildung 9

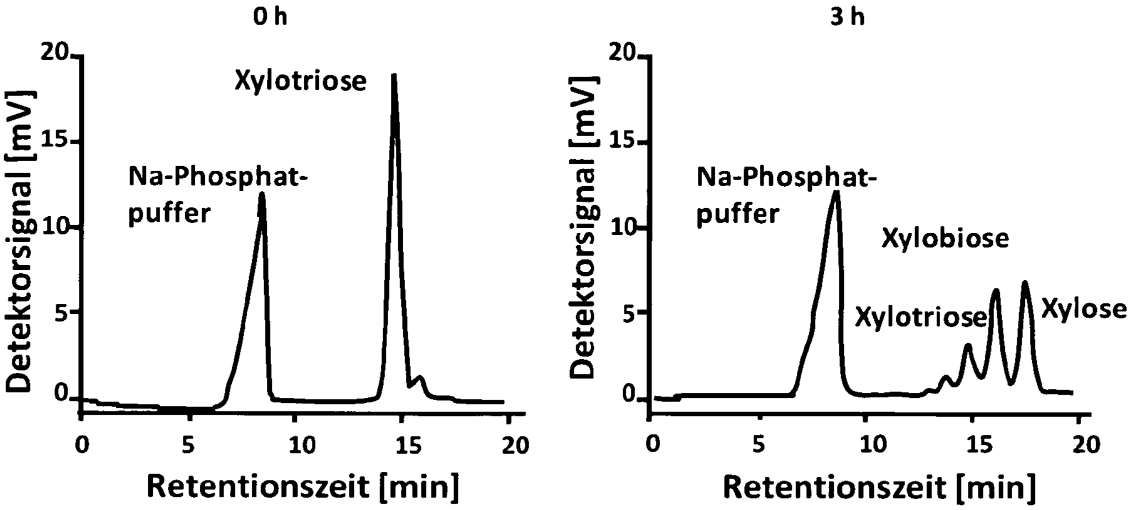


Abbildung 10

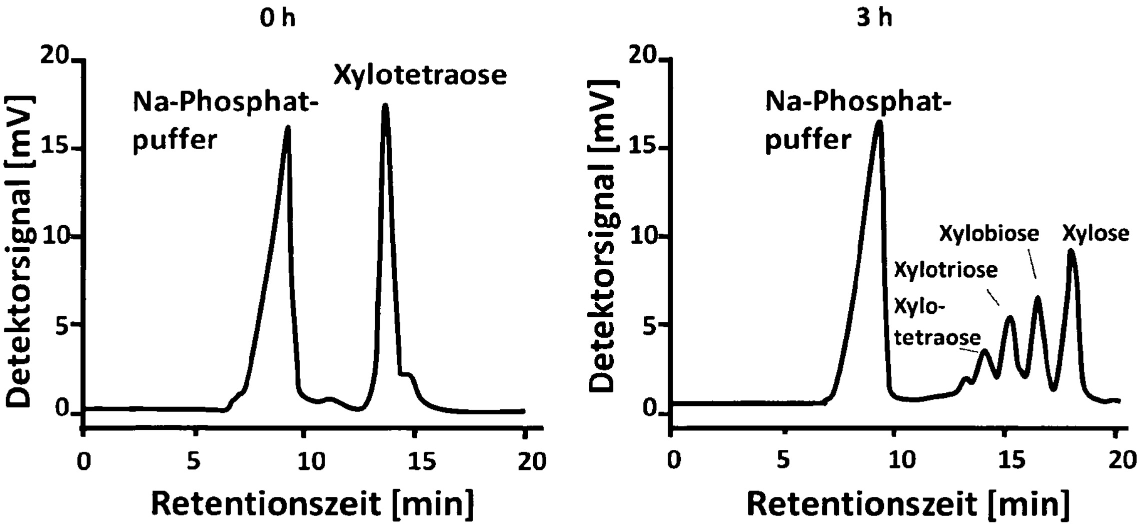


Abbildung 11

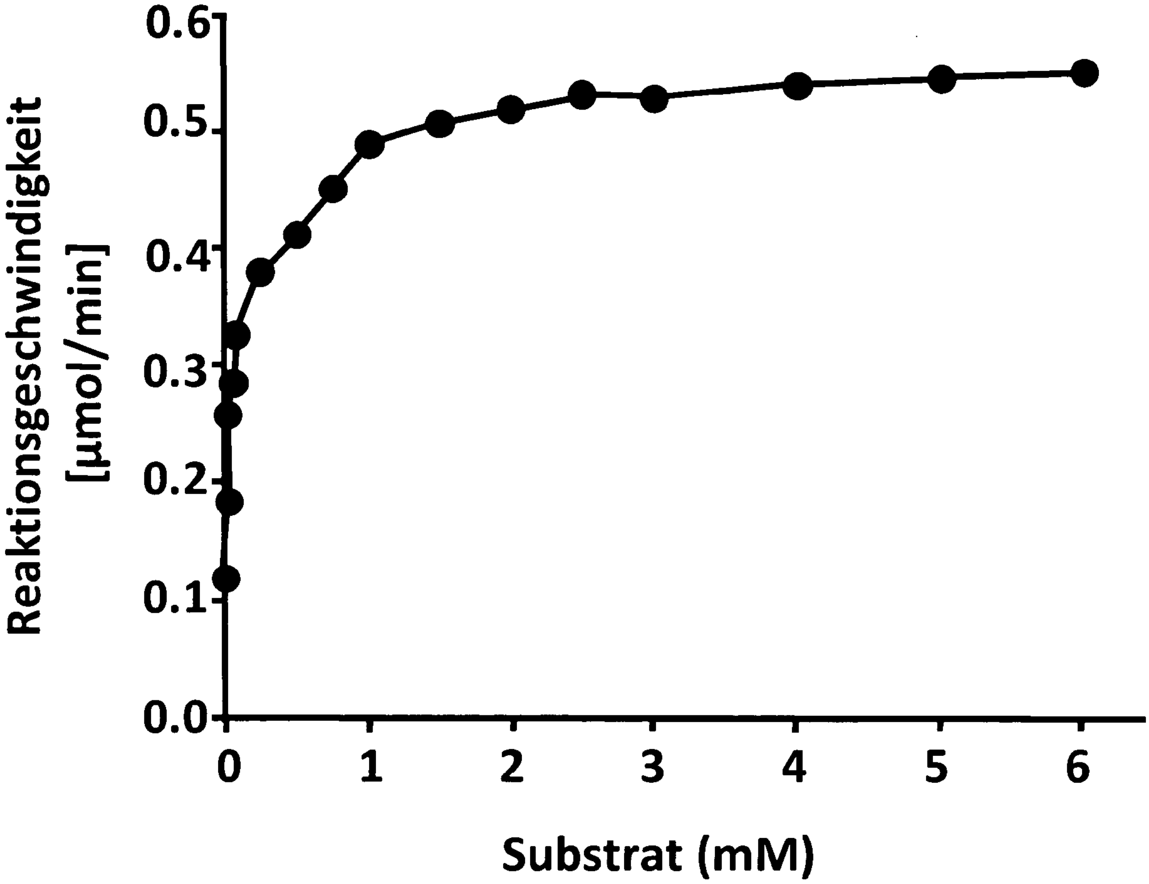


Abbildung 12

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 20091416464 A
- WO 2009094187 A
- US 3589585 A, Hollenberg


### In der Beschreibung aufgeführte Nicht-Patentliteratur


- **LYKO et al.** *J. Biotechnol.,* 2009, vol. 142 (1), 78-86
- **KUMAR et al.** *J. Ind. Microbiol. Biotechnol.,* 2008, vol. 35 (5), 377-391
- **PETERS.** *Adv. Biochem. Eng. Biotechnol.,* 2007, vol. 105, 1-30
- **KAMM et al.** *Biochem. Mol. Biol. Int.,* 2006, vol. 44 (2), 283-292
- **HENDRIKS ; ZEEMAN.** *Bioresour. Technol.,* 2009, vol. 100 (1), 10-18
- **TURNER et al.** *Microb. Cell. Fact.,* 2007, vol. 6, 9
- **VIIKARI et al.** *Adv. Biochem. Eng. Biotechnol.,* 2007, vol. 108, 121-145
- **ANTRANIKIAN G.** Industrial relevance of thermophiles and their enzymes - Thermophiles - Biology and Technology at high temperatures. CRC Press, 2008, 113-160
- **SAHA.** *J. Ind. Microbiol, Biotechnol.,* 2003, vol. 30 (5), 279-291
- **BERGQUIST et al.** *Methods Enzymol,* 2001, vol. 330, 301-319
- **KULKARNI et al.** *FEMS Microbiol. Rev.,* 1999, vol. 23 (4), 411-456
- **COLLINS et al.** *FEMS Microbiol. Rev.,* 2005, vol. 29 (1), 3-23
- **POLIZELI et al.** *Appl. Microbiol. Biotechnol.,* 2005, vol. 67 (5), 577-591
- **DWIVEDI et al.** *Appl. Microbiol. Biotechnol.,* 1996, vol. 45 (1-2), 86-93
- **JAEGER et al.** Biokatalyse. Angewandte Mikrobiologie. Springer Verlag, 2006, 135-160
- **HENRISSAT.** *Biochem. J.,* 1991, vol. 280 (2), 309-316
- **DAVIES ; HENRISSAT.** *Structure,* 1995, vol. 3 (9), 853-859
- **MCCARTER ; WITHERS.** *Curr. Opin. Struct Biol,* 1994, vol. 4 (6), 885-892
- **BORASTON et al.** *Biochem. J.,* 2004, vol. 382 (3), 769-781
- **HENRISSAT et al.** *FEBS Lett.,* 1998, vol. 425 (2), 352-354
- **JUNG et al.** *Biochem. Mol. Biol. Int.,* 1998, vol. 44 (2), 283-292
- **HAYASHI et al.** *J. Bacteriol.,* 1997, vol. 179 (13), 4246-4253
- **FONTES et al.** *Biochem. J.,* 1995, vol. 307 (1), 151-158
- **ZVERLOV et al.** *Appl. Microbiol. Biotechnol.,* 1996, vol. 45 (1-2), 245-247
- **SAUL et al.** *Appl. Environ. Microbiol.,* 1995, vol. 61 (11), 4110-4113
- **WINTERHALTER ; LIEBL.** *Appl. Environ. Microbiol.,* 1995, vol. 61 (5), 1810-1815
- **ADELSBERGER et al.** *Microbiology,* 2004, vol. 150 (7), 2257-2266
- **SHULAMI et al.** *J. Bacteriol.,* 1999, vol. 181 (12), 3695-3704
- **BREVES et al.** *Appl. Environ. Microbiol.,* 1997, vol. 63 (10), 3902-3910
- **LORENZ ; WIEGEL.** *J. Bacteriol.,* 1997, vol. 179 (17), 5436-5441
- **BEG et al.** *Appl. Microbiol. Biotechnol.,* 2001, vol. 56 (3-4), 326-338
- **BHAT.** *Biotechnol. Adv.,* 2000, vol. 18 (5), 355-383
- **GALANTE et al.** Enzymes, biological control and commercial applications. Verlag Taylor & Francis, 1998, vol. 2, 327-342
- **BHAT ; BHAT.** *Biotechnol. Adv.,* 1997, vol. 15 (3-4), 583-620
- Enzymes, biological control and commercial applications. **BUCHERT et al.** Trichoderma & Gliocladium. Verlag Taylor & Francis, 1998, vol. 2, 343-363
- **VARGHESE et al.** *Structure,* 1999, vol. 7 (2), 179-190
- *Nucleic Acid Research,* 1994, vol. 22 (22), 4673-4680
- **POZZO et al.** *J. Mol. Biol.,* 2010, vol. 397 (3), 724-739
- Molecular cloning, a laboratory manual. Cold Spring Harbor Laboratory Press, 1989
- **SAMBROOK et al.** Molecular cloning, a laboratory manual. Cold Spring Harbor Laboratory Press, 1989

- **SAMBROOK et al.** Molecular cloning, a laboratory manual. Cold Spring Harbor Laboratory Press
- **BENEYX.** *Curr. Opin. Biotechnol.,* 1999, vol. 10 (411), 422
- **HOTTINGER et al.** *Yeast,* 2004, vol. 10, 283-296
- **CEREGHINO et al.** *FEMS Microbiol. Reviews,* 2000, vol. 24, 45-66
- **PRICE et al.** *Methods Enzymol.,* 1990, 185-318
- **ALTSCHUL et al.** *Nucleic Acids Res,* 1997, vol. 25 (17), 3389-3402
- **ZVERLOV et al.** *Microbiology,* 1997, vol. 143 (11), 3537-3542
- **WULFF-STROBEL ; WILSON.** *J. Bacteriol.,* 1995, vol. 177 (20), 5884-5890
- **BRADFORD.** *Anal Biochem,* 1976, vol. 72, 248-254
- **LAEMMLI.** *Nature,* 1970, vol. 227 (5259), 680-685
- **PARK et al.** *Appl. Microbiol. Biotechnol.,* 2005, vol. 69 (4), 411-422